(19) Europäisches Patentamt
European Patent Office
Office européen des brevets

(11) Veröffentlichungsnummer: **0 165 437**
**B1**

(12) # EUROPÄISCHE PATENTSCHRIFT

(45) Veröffentlichungstag der Patentschrift: **21.09.88**

(21) Anmeldenummer: **85105673.9**

(22) Anmeldetag: **09.05.85**

(51) Int. Cl.⁴: **C 08 G 18/70**, C 07 C 118/00, B 01 J 13/02

(54) **Verfahren zur Herstellung von festen Polyisocyanaten retardierter Reaktivität, polymerumhüllte, feinteilige Polyisocyanate und ihre Verwendung.**

(30) Priorität: **18.05.84 DE 3418429**

(43) Veröffentlichungstag der Anmeldung: **27.12.85 Patentblatt 85/52**

(45) Bekanntmachung des Hinweises auf die Patenterteilung: **21.09.88 Patentblatt 88/38**

(84) Benannte Vertragsstaaten: **AT BE CH DE FR GB IT LI NL SE**

(56) Entgegenhaltungen:
**BE-A-651 504**
**DE-A-2 941 051**

(73) Patentinhaber: **BAYER AG, Konzernverwaltung RP Patentabteilung, D-5090 Leverkusen 1 Bayerwerk (DE)**

(72) Erfinder: **Grögler, Gerhard, Dr., von Diergardt-Strasse 46, D-5090 Leverkusen 1 (DE)**
Erfinder: **Kopp, Richard, Dr., Wolfskaul 12, D-5000 Koeln 80 (DE)**
Erfinder: **Hess, Heinrich, Dr., Körnerstrasse 5, D-5090 Leverkusen 1 (DE)**

**EP 0 165 437 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von festen, feinteiligen, durch eine Oberflächenhülle in ihrer Reaktivität retardierten Polyisocyanaten, dadurch gekennzeichnet, daß man

a) in einer ersten Stufe

- ein festes, feinteiliges Polyisocyanat A mit einem Schmelzpunkt oberhalb 38°C und einer Teilchengröße zwischen 0,5 und 200 µm mit

- einem in flüssiger Form vorliegenden, von A verschiedenen Polyisocyanat B, in einer Menge von 0,05 bis 50 Gew.-Teilen B pro 100 Teile A, in

- Suspension in einem Suspensionsmittel C, wobei C so ausgewählt ist, daß A nicht gelöst wird und B so schwer löslich ist, daß seine adsorptive Abscheidung auf A nicht verhindert wird, bei Temperaturen unterhalb des Schmelzpunktes von A oberflächlich bedeckt und danach gegebenenfalls C entfernt,

b) in einer zweiten Stufe

- das die Oberfläche der Polyisocyanatteilchen A bedeckende Polyisocyanat B mit mindestens einer mehrwertigen, in flüssiger Form vorliegenden, gegenüber Isocyanatgruppen reaktiven Verbindung D in Mengen von mindestens 0,5 Äquivalenten D pro Äquivalent B,

- gegebenenfalls in Suspension in einem Suspensionsmittel C, unter Ausbildung einer Hülle aus B und D um A umsetzt und

c) gegebenenfalls das Suspensionsmittel C entfernt.

Erfindungsgegenstand sind auch die nach dem erfindungsgemäßen Verfahren erhältlichen, umhüllten, feinteiligen Polyisocyanate mit retardierter Reaktivität.

Weiterer Erfindungsgegenstand ist die Verwendung der umhüllen Polyisocyanate, gegebenfalls in Suspension in höhermolekularen Polyolen und/oder höhermolekularen Polyaminen, zur Polyurethanherstellung, insbesondere in Einkomponenten-Reaktiv-Systemen hoher Lagerstabilität.

Bisher sind nur wenige Veröffentlichungen zur Oberflächenmodifizierung von bei Raumtemperatur festen Polyisocyanaten bekannt.

In der DE-A-2 557 407 wird ein Verfahren beschrieben, bei dem die Lösung eines Polyisocyanates in einem niedrig siedenden Lösungsmittel in einen Reaktionsraum mit einem gasförmigen Di- und/oder Polyamin gedüst wird, wobei durch die Reaktion des Polyisocyanats mit dem Amin und durch die Verdampfung des Lösungsmittels Polyurethanpolyarnstoff-Hohlperlen erhalten werden, die vorzugsweise als Füllstoffe eingesetzt werden. Die Reaktion wird so geführt, daß die NCO-Gruppen mit dem Amin und gegebenenfalls weiteren zugesetzten NCO-reaktiven Komponenten (z. B. Diolen) möglichst vollständig abreagieren.

US-A-3 409 461 beschreibt die Umhüllngung von Polyisocyanaten mit einer Schutzsubstanz, vorzugsweise einem Polymeren, wodurch die Polyisocyanatteilchen an der Oberfläche desaktiviert sind. Hierzu wird das Isocyanat in eine Lösung des Polymers in einem niedrig siedenden, das Isocyanat praktisch nicht lösenden Lösungsmittel dispergiert und die Dispersion sprühgetrocknet. Vorzugsweise wird fein gemahlenes (1 bis 10 µm Teilchengröße) Naphthylen-1,5-diisocyanat mit einer 1-bis 2,5 %-igen Lösung von Polystyrol, Polyvinylbutylether, chloriertem Kautschuk und ähnlichen in Tetrachlormethan sprühgetrocknet. Es werden freifließende Pulver mit einer Teilchengröße von ca. 1 bis 50 µm erhalten. Diese werden vorzugsweise zur Verbesserung der Haftung von Polyesterprodukten (Geweben, Fasern, Folien) an Kautschukelastomeren eingesetzt.

Bei diesem Verfahren zur Umhüllung von Isocyanaten mittels zugesetzter Polymerer aus Lösung müssen erhebliche Mengen von teilweise giftigen Lösungsmitteln eingesetzt werden, z. B. 4 kg Tetrachlormethan für 50 g Naphthylen-1,5-diisocyanat, die dann in einem energetisch aufwendigen Verfahren wieder entfernt werden müssen. Als besonderer Nachteil des Verfahrens muß der erhebliche Anteil des Hüllenmaterials an dem Gesamtgewicht des umhüllten Isocyanats angesehen werden. Dieser beträgt 9 bis 91 Gew.-%, bei den Beispielen liegt er im allgemeinen in der Größenordnung von 50 Gew.-%. Dadurch würde bei der Herstellung von qualitativ hochwertigen Polyurethanen ein zu hoher Anteil an störender Fremdsubstanz eingebracht werden.

Die US-A-3 551 346 beschreibt eine Verkapselung von flüssigen Diisocyanaten durch Grenzflächenreaktionen von im Diisocyanat gelösten $CH_3$-Si-$(OCH_3)_3$ mit in der Wasserphase gelöstem $(CH_3)_3$.Si-O-Na unter Filmbildung. Diese, durch Silikonpolymerbildung vorverkapselten Tröpfchen werden anschließend durch Coacervation (z. B. mit entgegengesetzt geladenen Polymeren nach US-A-2 800 457) endgültig "verkapselt".

In der DE-A-2 311 712 wird ein Verfahren zur Einkapselung von festen Stoffen durch eine Polymerhülle, gebildet aus NCO-Prepolymeren und Kettenverlangerungsmitteln, beschrieben, wobei man das reaktive Gemisch und eine wäßrige Phase bei einer Temperatur, bei der alle Reaktanten flüssig sind, in eine Zone hoher Turbulenz bringt und wobei sich unter Bildung eines hochmolekularen Polymeren als Umhüllungsmaterial (z. B. eines Polyharnstoffs aus NCO-Prepolymeren und Polyaminen) Mikrokapseln bilden. Mit Hilfe des Verfahrens können beliebige feste oder flüssige Stoffe eingekapselt werden, wenn sie gegenüber NCO-Prepolymeren und den Kettenverlängerungsmitteln (und Wasser) inert und wasserunlöslich sind, z. B. Trischlorethylphosphat-Flammschutzmittel, Weichmacher, Duftstoffe etc. Ähnliche Verfahren werden z. B. in der US-PS-4 120 518 für Einkapselungsreaktionen mit carbodiimidhaltigen Polyisocyanaten zur Bildung des Füllmaterials beschrieben.

In der DE-A-1 570 548 wird ein längere Zeit lagerfähiges Einkomponentsystem beschrieben, welches aus einem Gemisch von 1 Mol eines Polyesters, Polyethers oder Polythioethers, mindestens 1,5 Mol eines Uretdiongruppenenthaltenden festen Isocyanates mit einem Schmelzpunkt $\geqslant$100°C und mindesten 0,3 Mol

eines festen Kettenverlänogerungsmittels mit OH- und/oder $NH_2$-Gruppen mit einem Schmelzpunkt ≥80°C besteht. Dabei müssen mindestens 80 % der festen Gemischbestandteile mit einer Teilchengröße ≤38 µm vorliegen. Die Lagerbeständigkeit beträgt bei Raumtemperatur einige Tage bis einige Wochen, bei 50°C nur einige Stunden. Ein Nachteil des Verfahrens liegt darin, daß von drei Reaktionpartnern mindestens zwei in fester Form vorliegen müssen, um die nötige Lagerstabilität zu gewährleisten. Das führt dazu, daß im allgemeinen sehr hochviskose Gemische erhalten werden, deren Viskosität langsam weiter ansteigt, weil keine der Verbindungen in ihrer Reaktivität modifiziert wurde.

Die aus der ständigen Viskositätszunahme ersichtliche Reaktion an der Oberfläche der festen Teilchen erfolgt unkontrolliert und praktisch zu langsam und ergibt keine ausreichende Retardierung der Reaktivität der Polyisocyanate. Auch dürften beim Aushärten der Mischung wegen der hohen Anteile an festen Bestandteilen Inhomogenitäten im ausgeheizten Produkt zu erwarten sein. Ferner ist die Verarbeitung der hochviskosen bis festen Gemische schwierig, weil diese im Gegensatz zu flüssigen Gemischen zuerst einmal durch Temperaturerhöhung oder Druckanwendung in einem verformbaren Zustand gebracht werden müssen.

Wie sich in Vergleichsversuchen gezeigt hat, tritt beim Verweilen von hochschmelzenden Polyisocyanaten in Gemischen aus höher- und niedermolekularen Polyolen eine ständige und relativ schnelle Weiterreaktion unter starker Viskositätserhöhung ein, d.h. die Oberflächenreaktion an den festen Polyisocyanatteilchen bildet zumeist keine zur Retardierung ausreichende, d. h. hinreichend gegen eine weitere Reaktion innerer Schichten schützende Hülle um die Polyisocyanatteilchen.

In der GB-A-1 134 285 wird ein Verfahren zur Herstellung von dimeren Diisocyanaten in einem wäßrigen Reaktionsmedium beschrieben. Derartig hergestellte Dimere in wäßriger Suspension reagieren nach den Angaben in dieser Patentschrift mit polyfunktionellen Verbindungen mit aktiven Wasserstoffatomen nicht bei Raumtemperatur, können jedoch thermisch zu Polyurethanen vernetzt werden. Möglicherweise wird die Lagerstabilität bei Raumtemperatur durch eine eingetretene, langsame Oberflächenreaktion von Isocyanatgruppen mit Wasser hervorgerufen. Eine Vernetzung wird anschließend bei hohen Temperaturen, z. B. 150 bis 200°C, durch Spaltung des Uretdionrings herbeigeführt.

Lagerstabile Reaktivsytme mit langer Topfzeit werden auch in den DE-A-2 842 805 und 2 941 051 beschrieben, wobei Polyisocyanatgemische aus hochschmelzenden Polyisocyanaten (z. B. dimerem TDI) und flüssigen Polyisocyanaten in höhermolekularen und niedermolekularen Polyolen, gegenenfalls in Gegenwart unterschüssiger Mengen einer Verbindung mit zwei bis vier Aminogruppen, z. B. cyloaliphatischen Di- oder Triaminen, Hydrazin und substituierten Hydrazinen oder Säurehydraziden unter Bildung einer stabilen Dispersion vermischt und gegebenenfalls nach Zusatz von Glasfasern in einer zweiten Stufe oberhalb 90°C unter Formgebung ausgehärtet werden.

Mit unterschüssigen Mengen (0,01 bis 20 bzw. 25 % der vorhandenen NCO-Gruppen) an Diaminen und anderen NH-funktionellen Verbindungen umgesetzte feste Polyisocyanate in Mischung mit Polyolen, Polyaminen oder Polyhydraziden werden auch in der DE-A-3 112 054, 3 228 723, 3 228 724 und DE-A-3 230 757 beschrieben. Die Polyisocyanatteilchen sind somit an ihrer Oberfläche durch Reaktion von bis zu 25 Äquivalent % der insgesamt vorhandenen NCO-Gruppen unter Bildung einer Polymer (z. B. Polyharnstoff)hülle desaktiviert. Dispersionen solcher desaktivierter Polyisocyanatteilchen in Polyolen und/oder Polyaminen zeigen zumindest bei Raumtemperatur eine sehr gute Lagerstabilität. Erst oberhalb einer Verfestigungs -oder Aufdickungstemperatur geht das Reaktivsystem die polyurethan(harnstoff)bildenden Reaktionen ein und bildet dann Elastomere oder ähnliche Endprodukte.

Wie bereits in der DE-A-3 230 757 festgestellt wurde, reagieren die für die oberflächliche Umhüllungsreaktion eingesetzten Verbindungen, z. B. die aliphatischen Polyamine, zumeist - und sogar weitgehend unabhängig von der Aminmenge - nicht vollständig ab, wenn sie in Dispersion in Polyolen oder Polyaminen umgesetzt werden. Somit verbleibt ein Teil dieser Polyamine unreagiert in der PU-Reaktiv-Dispersion. Dies kann bisweilen einen erwünschten Effekt bewirken, indem bei der Lagerung oder Handhabung der Reaktivmischung eventuell eintretende Oberflächendefekte in der Polyharnstoffumhüllung durch Eindringen der Polyamine wieder geheilt werden ("Selbstheileffekt") und somit die Lagerstabilität weiter gegeben ist.

Andererseits aber bringt dieser Effekt einen großen Verfahrensnachteil mit sich, wenn man größere (großvolumige) Formkörper aus solchen, in situ desaktivierten Polyisocyanat/Polyol- oder Polyamin-Mischungen als Einkomponentenreaktivsystem herzustellen versucht. Es hat sich nämlich gezeigt, daß beim Aufschmelzen solcher Reaktivsysteme eine unkontrollierte weiter Desaktivierung des Polyisocyanats (vor allem in Inneren des Formkörpers) mit noch nicht umgesetzten Polyamin stattfindet und somit die Aufdickungstemperatur im Kern ständig weiter ansteigt. Dadurch können Lunker entstehen, bzw. kann auch die Oberfläche gestört werden. Die Festigkeit solcher inhomogen aufgebauter Formkörper ist dann unbefriedigend (siehe Beispiele und Vergleichsversuche).

Ein weiterer Nachteil des bekannten Verfahrens liegt darin, daß bei einem Entfernen der in der Dispersion verbleibenden, nicht umgesetzten Aminkomponente die Lagerstabilität der Dispersionen oftmals deutlich erniedrigt wird oder sogar gänzlich verloren geht (siehe Vergleichsbeispiele).

Aus diesem Grund ist es z. B. nicht möglich, ein festes Polyisocyanat in gegen Isocyanatgruppen reaktionsfähigen Verbindungen (z. B. in Polyolen) nach dem Stand der Technik durch oberflächliche Umsetzung der Polyisocyanatteilchen, z. B. mit einem aliphatischen Diamin, zu umhüllen und anschließend diese gegen Isocyanatgruppen reaktionsfähigen Verbindungen durch Reaktion mit üblichen flüssigen Polyisocyanaten umzusetzen (beispielweise, um eine Vorverlängerung eines langkettigen Polyols zu erreichen,

wie es zum Umgehen von Viskositätsproblemen notwendig sein kann, oder um eine Thixotropierung zu ereichen), Das zugesetzte flüssige Polyisocyanat bewirkt nämlich eine Destabilisierung der Dispersion (siehe Beispiele und Vergleichsbeispiele).

Auch im Falle der Verwendung von aliphatischen Diaminen für die Umhüllung ist der Zusatz von gegenüber aliphatischen Diaminen reaktionsfähigen oder diese adsorbierenden Stoffen, wie z. B. halogenhaltigen Flammschutzmitteln und Treibmitteln, Zeolithen, Fettsäuren, Phosphorestern oder bestimmten Lösungsmitteln störend oder gar unmöglich.

Ein weiterer Nachteil dieser bekannten Verfahren liegt darin, daß bei der Umhüllung bis zu 25 Äquivalent % der festen, im allgemeinen sehr teuren Polyisocyanatkomponente verbraucht werden und für weitere Reaktionen nicht mehr zugänglich sind.

Aufgabe der vorliegenden Erfindung war es nun, Polyisocyanatteilchen retardierter Reaktivität herzustellen, deren NCO-Gruppen noch im wesentlichen unverändert vorliegen und die trotzdem eine Schutzhülle aufweisen, welche eine sichere Retardierung der Reaktivität bewirkt. Hiermit sind dann auch reaktive Mischungen mit hoher Lagerstabilität und trotzdem guter z. B. thermisch ausgelöster Polyurethanbildung, zusätzlich. Ferner lassen sich Reaktivsystme erhalten, die keine ständige Zunahme der Aufdickungstemperatur bei der Herstellung von großvolumigen Formkörpern zeigt. Weitere Aufgaben der Erfindung sind in Beschreibung und Beispiele dargestellt oder ersichtlich.

Diese Aufgabe wird mit dem Verfahren gemäß Patentanspruch 1 gelöst.

Erfindungsgegenstand sind auch die nach dem erfindungsgemäßen Verfahren erhältlichen, umhüllten, feinteiligen Polyisocyanate mit retardierter Reaktivität, gegebenenfalls suspendiert in höhermolekularen Polyolen und/oder höhermolekularen Polyaminen, und gegebenenfalls niedermolekularen Polyolen und/oder aromatischen Diaminen.

Suspensionen der umhüllten Polyisocyanate in höhermolekularen Polyolen sind bevorzugt. Sie können erfindungsgemäß auch dadurch erhalten werden, daß man die festen Polyisocyanate A in Polyolen suspendiert und mit anderen Polyisocyanaten in flüssiger Form, d.h. mit flüssigen gelösten oder emulierten Polyisocyanaten B oberflächlich bedeckt und dann mit D umsetzt.

Ein weiterer Erfindungsgegenstand ist die Verwendung der erfindungsgemäß erhältlichen, umhüllten Polyisocyanate, gegebenfalls in Suspension in Polyolen und/oder Polyaminen und/oder Weichmachern, zur Polyurethanherstellung, bevorzugt in Einkomponenten-Reaktivsystemen mit hoher Lagerstabilität und erhöhter Aufdickungstemperatur. Solche Systeme können durch Erwärmung und/oder Zufuhr mechanischer Energie und/oder mittels polaren Lösungsmitteln aktiviert werden und insbesondere zur Herstellung von Elastomeren , zelligen Polyurethanen, Fugenvergußmassen und Verklebungen Anwendung finden.

Bei dieser Verwendung der erfindungsgemäßen umhüllten Polyisocyanate können gegebenfalls auch niedermolekulare Kettenverlängerungsmittel, Polyurethankatalysatoren und übliche Hilfs- und Zusatzstoffe, sowie weitere, nicht umhüllte Polyisocyanate zugesetzt werden.

Die Reaktivsysteme sind fließfähig oder leicht aufschmelzbar, haben eine Aufdickungstemperatur von ⩾55°C und ihre Aushärtung kann durch Einwirkung von Hitze, Scherkräften und/oder polaren Lösungsmitteln zu massiven oder zelligen Polyurethanen erfolgen.

Die erfindungsgemäßen Polyisocyanate und Reaktivmischungen zeigen nicht die bereits angeführen Nachteile nach dem Stand der Technik. Die Erfindung vermeidet die Nachteile des aufgezeigten Standes der Technik dadurch, daß zur Umhüllung der festen Polyisocyanatteilchen A nicht deren NCO-Gruppen verbraucht werden, sondern daß die Umhüllung durch Reaktion eines anderen Polyisocyanats B, welches an der Oberfläche von A adsorbiert wurde, mit einer gegen Isocyanatgruppen reaktiven Verbindung gebildet wird. Dieses Polyisocyanat B wird dabei zunächst als "Bedeckung" auf das feste Polyisocyanat A aufgebracht und dort adsorbiert. Die Hülle besteht aus dem Reaktionsprodukt (vorzugsweise einem Polymer) aus B/D in Gegensatz zu der bekannten Polymerumhüllung durch einen Polyharnstoff aus A/D.

Da die beiden Komponenten B/D wegen ihres nicht festen Aggregatzustands vollständig abreagieren, treten keine Probleme infolge einer nachträglichen Veränderung der Hülle um das feste Polyisocyanat auf. Das heißt, die Aufdickungstemperatur bleibt auch bei langsamem Aufheizen bei erhöhten Temperaturen und auch bei der Herstellung großvolumiger Formkörper konstant (siehe Beispiele).

Wie die bisherigen Untersuchungen zeigen, ist erfindungsgemäß der Aufdickungspunkt im allgemeinen von der Menge der gebildeten Polyharnstoffhülle in nicht so starken Maße abhängig, wie dies bei der Umhüllung mittels eines aliphatischen Polyamins nach dem Stand der Technik der Fall ist (siehe Beispiele und Vergleichsbeispiele).

In den erfindungsgemäßen Reaktivsystemen sind weitere Reaktionen mit allen in der Polyurethanchemie üblichen Komponenten möglich. Zum Beispiel kann durch Zugabe von flüssigen Diisocyanaten und Katalysatoren eine Verlängerung eines im Reaktivsystem enthaltenen Polyols ( z. B. Polyetherpolyols) vorgenommen werden (siehe Beispiele).

Es können auch Hilfsstoffe und Additive zugesetzt werden, die bei analogen lagerstabilen Systemen nach dem Stand der Technik nicht eingesetzt werden können, z. B. halogenhaltige Flammschutzmittel, Säureanhydride, Fettsäuren, Carbodiimide, Phosphate und Phosphite oder Zeolithe.

Überraschenderweise bildet sich die Umhüllung besonders günstig mit aliphatischen Polyisocyanaten als Komponente B, obwohl die Reaktivität der aliphatischen NCO-Gruppen geringer ist als die aromatischer NCO-Gruppen.

Als feste, feinteilige Polyisocyanate A sind alle Di- oder Polyisocyanate oder deren beliebige Gemische

geeignet, sofern sie einen Schmelzpunkt oberhalb 38°C, vorzugsweise oberhalb 70°C, besonders bevorzugt oberhalb 110°C, aufweisen.

Es können dies aliphatische, cycloaliphatische, araliphatische, bevorzugt aber aromatische und heterocylische Polyisocyanate, sein, ferner Polyphenyl-polymethylenpolyisocyanate, erhalten durch Anilin-Formaldehyd-Kondensation und anschließende Phosgenierung nach den britischen Patentschriften 874 430 und 848 671, weiterhin perchlorierte Arylpolyisocyanate, Carbodiimidgruppen aufweisende Polyisocyanate, Allophanatgruppen aufweisende Polyisocyanate, Isocyanuratgruppen aufweisende Polyisocyanate, Urethan- oder Harnstoffgruppen aufweisende Polyisocyanate, acylierte Harnstoffgruppen aufweisende Polyisocyanate, Biuretgruppen aufweisende Polyisocyanate, durch Telomerisationsreaktion hergestellte Polyisocyanate, Estergruppen aufweisende Polyoisocyanate, ferner bevorzugt Uretdiongruppen aufweisende Diisocyanate und Harnstoffgruppen aufweisende Diisocyanate. Als Beispiel für die einsetzbaren Polyisocyanate seien genannt:

| | |
|---|---|
| 1,5-Diisocyanatomethylnaphthalin | Fp.: 88 - 89°C |
| 1,4-Phenylendiisocyanat | 94 - 96°C |
| 1,3-Dimethylbenzol-4,6-diisocyanat | 70 - 71°C |
| 1,4-Dimethylbenzol-2,5-diisocyanat | 76°C |
| 1,4-Dichlorbenzol-2,5-diisocyanat | 134 - 137°C |
| 1-Methoxybenzol-2,4-diisocyanat | 75°C |
| 1-Methoxybenzol-2,5-diisocyanat | 89°C |
| 1,3-Dimethoxybenzol-4,6-diisocyanat | 125°C |
| Azobenzol-4,4'-diisocyanat | 158 - 161°C |
| Diphenylether-4,4'-diisocyanat | 66 - 68°C |
| Diphenyl-dimethylmethan-4,4'-diisocyanat | 92°C |
| Naphthalin-1,5-diisocyanat | 127 - 130°C |
| 1,4-Bis(1'-methyl-1'-isocyanato)ethyl-benzol | 72°C |
| Tetramethyl-p-xylylendiisocyanat | |
| 3,3'-Dimethylbiphenyl-4,4'-diisocyanat | 68 - 69°C |
| Diphenylsulfon-4,4-diisocyanat | 154°C |
| 4,4'-Diisocyanato-(1,2)-diphenyl-ethan | 88 - 90°C |
| dimeres 1-Methyl-2,4-phenylendiisocyanat | 155°C |
| dimeres 1-Isopropyl-2,4-phenylendiisocyanat | 125°C |
| dimeres 1-Chlor-2,4-phenyldiisocyanat | 177°C |
| dimeres 2,4'-Diisocyanatodiphenylsulfid | 178 - 180°C |

dimeres Diphenylmethan-4,4-diisocyanat
3,3-Diisocyanato-4,4' (oder 2,2)-dimethyl-N,N-diphenylharnstoff
N,N'-Bis[4(4-Isocyanatophenylmethyl)phenyl]harnstoff
N,N'-Bis [4(2-Isocyanatophenylmethyl)phenyl]harnstoff
trimeres 2,4-Diisocyanatotoluol(isocyanurat-isocyanat)
trimeres 4,4'-Diisocyanatodiphenylmethan (isocyanurat-isocyanat)
trimeres Isophorondiisocyanat(isocyanurat-isocyanat) (vgl. DE-A-2 806 731)
oder das Addukt von 3 Mol 2,4-Diisocyanatotoluol mit 1Mol Trimethylolpropan.
Ferner sind Dimer-harnstoffdiisocyanate entsprechend der DE-A-3 232 736 geeignet.
Ferner sind Mischtrimerisate auf Basis von z. B. TDI/MDI, TDI/HDI (vgl. DE-A-3 033 860) oder Produkte entsprechend DE-A-3 041 732 geeignet, soweit sie feste Produkte mit entsprechendeb Schmelzpunkten darstellen.

Erfindungsgemäß werden bevorzugt dimere und trimere Diisocyanate, sowie Harnstoffdiisocyante, z. B. 3,3'-Diisocyanato-4,4'-dimethyl-N,N'-diphenylharnstoff, dimeres 2,4-Diisocyanatotoluol, dimeres 4,4'-Diisocyanatodiphenylmethan und 3,3'-Dimethyl-4,4'-diisocyanatodiphenylmethan, sowie die Isocyanurate (Trimere) auf Basis von 2,4-Diisocyanatotoluol, eingesetzt. Die dimeren und trimeren Diisocyanate können auch durch in situ-Dimerisierung oder Trimerisierung bzw. die Harnstoffdiisocyanate durch Wasserreaktion in z. B. Weichmachern, Lösungsmitteln oder Polyolen feinverteilt hergestellt und in dieser Form besonders einfach (weil ohne vorherige Isolierung und ohne mechanische Zerkleinerung) umhüllt werden. Die Teilchengröße von A liegt zwischen 0,5 und 200 µm, vorzugsweise 1 bis 50 µm.

Als Polyisocyanate B zur "Bedeckung" der Oberfläche der festen, feinteiligen Polyisocyanate A werden von A unterschiedliche Polyisocyanate in flüssiger Form eingesetzt. Es sind dies vorzugsweise flüssige, ölige oder harzartige Diisocyanate oder insbesondere höherfunktionelle Polyisocyanate mit >2 NCO-Gruppen. Bevorzugt sind (cyclo)aliphatische di- und insbesondere höherfunktionelle Polyisocyanate, insbesondere biuretisierte, dimerisierte oder trimerisierte, oder mit Polyolen modifizierte Polyisocyanate (z. B. mit höhermolekularen Polyolen hergestellte NCO-Prepolymere). Auch Gemische der genannten Polyisocyanate B sind einsetzbar.

Beispiele sind Hexamethylendiisocyanat, Undecamethylendiisocyanat , Dodecamethylendiisocyanat, Dimersäure-di und -polyisocyanate, Isophorondiisocyanat, Cylcohexandiisocyanate,α,α,α',α',-Tetramethyl-m/p-hexahydroxylylen-diisocyanate, 4-Isocyanatomethyl-octan-1,8-diisocyanat, Hexahydrotoluylendiisocyanate, Dicyclohexylmethan-diisocyanate, 1,6,11-Triisocyanatoundecan, substituierte 1,5-Diisocyanatopentanderivate entsprechend EP-A-77 105 (z. B. 1-Methyl-1,5-pentandiisocyanat) oder ihre oligomeren Derivate (z. B.

entsprechend EP-A-77 104).

Besonders bevorzugt sind flüssige oder ölige Polyisocyanate mit mehr als zwei NCO-Gruppen, z. B. biuretisierte, carbodiimidisierte, trimerisierte oder polyolmodifizierte Polyisocyanate, z. B. auf Basis Hexamethylendiisocyanat, Isophorondiisocyanat oder Dicyclohexylmethandiisocyanat.

Ganz besonders günstig sind biuretisiertes Hexamethylendiisocyanat (z. B. Desmodur®N, BAYER AG, Leverkusen). Aromatische Diisocyanate, z. B. TDI oder MDI sind weniger günstig, jedoch sind die Trimerisate von TDI oder insbesondere NCO-Prepolymere, z. B. von Trimethylolpropan und TDI in Form konzentrierter Lösungen in z. B. Toluol oder in Form von Emulsionen in Toluol/Petrolethermischungen, gut einsetzbar, da sie sich hieraus adsorptiv gut auf den festen, feinteiligen Polyisocyanaten A, z. B. dimerem Toluylendiisocyanat, abscheiden.

Als Suspensionsmittel C) finden vorzugsweise wenig polare, insbesondere (cyclo)aliphatische Kohlenwasserstoffe (z. B. Petroletherfraktionen, Benzin oder Cyclohexan) sowie längerkettige Dialkylether, z. B. Diisopropylether, Diisobutylether oder Diisooctylether, Verwendung.

Höherpolare Lösungsmittel, z. B. aromatische Kohlenwasserstoffe wie Toluol sind in den meisten Fällen als alleinige Suspensionsmittel nicht geeignet, doch können sie zur Herstellung von Emulsionen von B, z. B. durch Vermischen von Toluol-Lösungen von B mit Petrolether o.ä. verwendet werden.

Es hat sich gezeigt, daß aus z. B. Petrolether- bzw. Hexanlösungen die Polyisocyanate B adsorptiv oder auch nach (teilweisem) Abdampfen der Lösungsmittel leicht auf A niedergeschlagen werden. Man dispergiert daher vorzugsweise die festen Polyisocyanate A in aliphatischen Kohlenwasserstoffen oder den genannten Ethern und fügt z. B. die Polyisocyanate B, gelöst in einer kleinen Menge an polareren Lebensmitteln, wie Toluol, zu, wobei B zumeist ölig in den dispergierenden, aliphatischen Kohlenwasserstoffen ausfällt und auf A adsorptiv als Bedeckung niedergeschlagen wird. Je nach Löslichkeit von B können durch einfache Vorversuche solche Lösungsmittel bzw. Lösungsmittelgemische als Suspensionsmittel C ausgewählt werden, welche eine Bedeckung von A mit B erlauben. Die Suspensionsmittel müssen demnach so gewählt werden, daß zwar B zunächst in eine gelöste oder emulgierte Phase überführt wird, A jedoch (im wesentlichen) ungelöst und feinteilig dispergiert bleibt und B andererseits so schwer löslich ist, daß seine adsorptive Abscheidung auf A nicht verhindert wird. Die Menge an C wird vorzugsweise auf ein Minimum derart begrenzt, daß feste oder harzartige Polyisocyanate B eben gelöst oder ölig emulgiert werden, die feinteiligen Polyisocyanate A jedoch nicht gelöst werden, und daß eine rührbare Suspension von A/B/C bei der Umsetzung mit D entsteht. Die Menge an G beträgt im allgemeinen ca. 5 - 75 Gew.-%, bezogen auf (A + B + C), vorzugsweise 10 - 50 Gew.-%.

Die Verwendung von (höhermolekularen) Polyolen als Komponente C ist bevorzugt, da die durch anschließende Reaktion mit D entstandene Suspension von umhüllten Polyisocyanatteilchen in diesen Polyolen dann direkt zur weiteren Umsetzung zu Polyurethanen benutzt werden kann. Die maximale Polyolmenge wird in diesem Fall durch die Äquivalenz zu den NCO-Gruppen der vorhandenen Isocyanate begrenzt, um zu einem hochpolymeren Aufbau zu kommen, d.h. etwa 1 : 1 (in Praxis ca. 0,95 bis 1,10 : 1) aktive H-Atome: NCO.

Gegebenenfalls ist es nach dem Zusammentreffen von festem, zu umhüllenden Polyisocyanat A und dem zur Umhüllung eingesetzten Polyisocyanat B erforderlich, eine gewisse Wartezeit einzuhalten, bis das in flüssiger Form vorliegende Polyisocyanat B auf dem festen Polyisocyanat A adsorbiert ist (siehe Beispiele). Bei diesen Vorgängen spielt das Suspensionsmittel C eine wesentliche Rolle. Vorteilhaft sind unpolare Suspensionsmittel wie die genannten aliphatischen Kohlenwasserstoffe oder Ether, wie Diisopropylether, lipophile Weichmacher oder Polypropylenglykolpolyether, in denen das flüssige Polyisocyanat B relativ schwer löslich ist und sich dadurch schneller auf dem festen Polyisocyanat A niederschlägt.

Die eingesetzten Mengen B/A betragen 0,05 bis 50 Gew.-Teile B pro 100 Gew.-Teile A, bevorzugt 0,2 bis 25, insbesondere 1 bis 12 Gew.-Teile/100 Gew.-Teile A.

Man kann die Bedeckung auch direkt in Weichmachern, z. B. Adipinsäuredialkylestern oder Trialkylposphaten, insbesondere langkettigen Trialkylestern wie Stearylester, durchführen. Dabei können auch Anteile an leichtflüchtigen, wenig polaren Lösungsmitteln (aliphatische Kohlenwasserstoffe) mitverwendet und nach der Bedeckung oder nach der Umhüllungsreaktion gegebenenfalls wieder entfernt werden.

Insbesondere zur Herstellung von Einkomponenten-Reaktivmischungen können die Polyisocyanate A auch direkt in Polyolen dispergiert und mit B (gegebenenfalls unter Zusatz inerter Lösungsmittel, welche später entfernt werden) auf der Oberfläche bedeckt werden.

Die mit B bedeckten, feinteiligen Polyisocyanate A stellen - nach Entfernung des Suspensionsmittels C - im allgemeinen gut rieselfähige, nicht klebende Feststoffpulver dar, die sich gut handhaben lassen.

Als Komponente D können folgende Verbindungsklassen eingesetzt werden:

1. aliphatische und cycloaliphatische Di- und Polyamine;
2. Hydrazin, Alkylhydrazine, N,N'-Dialkylhydrazine, Di- und Polyhydrazidverbindungen;
3. offenkettige, mono- oder bicyclische Amidine und Guanidine ohne gegenüber NCO-Gruppen reaktiven H-Atomen, wie sie in DE-A-3 403 500 (EP-A-150 790) beschrieben werden;
4. mono- oder bifunktionelle Amidine und Guanidine mit einem oder zwei gegenüber NCO-Gruppen reaktiven H-Atomen;
5. Di- und/oder Triole mit Molekulargewichten 62 - 399

und deren Mischungen.

Geeignete Di- und Polyamine sind z. B. Ethylendiamin, 1,2- und 1,3-Propandiamin, 1,4-Butandiamin, 1,6-Hexandiamin, Neopentandiamin, 2,2,4- und 2,4,4-Trimethyl-1,6-diaminohexan, 2,5-Dimethyl-2,5-diaminohexan, 1,10-Decandiamin, 1,11-Undecandiamin, 1,12-Dodecandiamin, Bis-Aminomethyl-hexahydro-4,7-methanoindan

(TCD-Diamin), 1,3-Cyclohexandiamin, 1,4-Cyclohexandiamin, 1-Amino-3,3,5-trimethyl-5-aminomethyl-cyclohexan (Isophorondiamin), 2,4- und/oder 2,6-Hexahydrotoluylendiamin, 2,4'- und/oder 4,4'-Diaminodicyclohexylmethan, m- oder p-Xylylendiamin, Bis (3-aminopropyl) methylamin, Bis-N,N'-(3-aminopropyl)-piperazin und 1-Amino-2-aminomethyl-3,3,5-(3,5,5)-trimethylcyclopentan, 2,2-Dialkylpentan-1,5-diamine oder Triamine wie 1,5,11-Triaminoundecan, 4-Aminomethyl-1,8-diaminooctan, Lysinmethylester, cycloaliphatische Triamine gemäß DE-A-2 614 244, 4,7-Dioxadecan-1,10-diamin, 2,4- und 2,6-Diamino-3,5-diethyl-1-methylcyclohexan und deren Gemische, alkylierte Diaminodicylohexylmethane, z. B. 3,3'-Dimethyl-5-5'-diamino-dicylohexylmethan oder 3,5-Diisopropyl-3',5'-diethyl-4,4'-diaminodicyclohexylmethan, perhydrierte Diaminonaphthaline, perhydrierte Diaminoanthrazene, oder höherwertige Amine wie Diethylentriamin, Triethylentetramin, Pentaethylenhexamin, Dipropylentriamin, Tripropylentetramin, oder N,N'-Dimethylethylendiamin, 2,5-Dimethylpiperazin, 2-Methylpiperazin, Piperazin-(hydrat), 2-Hydroxyethylpiperazin und α,α,α',α'-Tetramethyl-m/p-xylylendiamin.

Außer diesen niedermolekularen aliphatischen Diaminen oder im Gemisch hiermit können auch höhermolekulare aliphatische Di- und Polyamine verwendet werden, wie sie z. B. durch reduktive Aminierung von Polyoxyalkylenglykolen mit Ammoniak nach BE-A-634 741 oder USA-3 654 370 erhalten werden können. Weitere höhermolekulare Polyoxyalkylen-Polyamine können nach Methoden, wie sie in der Firmenschrift "Jeffamine, Polyoxypropylene Amines" von Texaco Chemical Co., 1978, aufgezählt werden, hergestellt werden, beispielsweise durch Hydrierung von cyanethylierten Polyoxypropylenglykolen (DE-A-1 193 671), durch Aminierung von Polyproylenglykolsulfonsäureestern (US-A-3 236 895), durch Behandlung eines Polyoxyalkylenglykols mit Epichlorhydrin und einem primären Amin (FR-A-1 466 708) oder durch Umsetzung von NCO-Prepolymeren mit Hydroxylgruppen aufweisenden Enaminen, Aldiminen oder Ketiminen und anschließend Hydrolyse gemäß DE-B-2 546 536. Geeignete höhermolekulare aliphatische Di- und Polyamine sind auch die nach DE-A-2 948 419 und DE-A-3 039 600 durch alkalische Hydrolyse von NCO-Prepolymeren (mit aliphatischen Diisocyanaten) mit Basen über die Carbamatstufe zugänglichen Polyamine. Diese höhermolekularen Polyamine besitzen Molekulargewichte von etwa 400 bis 6000, vorzugsweise 400 bis 3000 und besonders bevorzugt von 1000 bis 3000. Infolge ihres Aufbaus sind derartige höhermolekulare Polyamine zur Ausbildung einer mechanisch stabilen Polyharnstoffumhüllung besonders geeignet. Sie werden daher, vorzugs weise in Mischung mit niedermolekularen Di- und Polyaminen, zur Umhüllung der Polyisocyanatteilchen A eingesetzt.

Als Komponente D sind auch Hydrazin, Alkylhydrazine sowie N,N'-Dialkylhydrazine vorzugsweise mit $C_1$-$C_6$-Alkylgruppen, die auch als weitere Substituenten Chlor- oder OH-Gruppen tragen können (mit Molekulargewichten von vorzugsweise 32 bis 198) und/oder zwei- oder mehrfunktionelle, nieder- oder höhermolekulare Verbindungen mit -CO-NH-$NH_2$-Endgruppen, und einem Molekulargewicht von 90 bis etwa 6000, vorzugsweise 90 bis 3000, geeignet. Es sind dies z. B. Hydrazin, zumeist in Form von Hydrazinhydrat, alkylsubstituierte Hydrazine, z. B. Methylhydrazin, Ethylhydrazin, Hydroxyethyl-hydrazin oder N,N'-Dimethylhydrazin. Weiter geeignete Verbindungen mit Hydrazid-Endgruppen sind z. B. Di- oder Polyhydrazide wie Carbodihydrazid, Hydracrylsäurehydrazid, Oxalsäuredihydrazid, Adipinsäuredihydrazid, Terephthalsäuredihydrazid, Isophthalsäurehydrazid oder Verbindungen mit Hydrazid- und Semicarbazid-, Carbazin-ester- oder Amino-Gruppen, z. B. β-Semicarbazidopropionsäurehydrazid, 2-Semicarbazidoethylcarbazinester, Aminoessigsäurehydrazid, β-Aminopropionsäurehydrazid oder Biscarbazinester oder Bissemicarbazide wie Ethylen- bis-carbazinester bzw. Ethylen-bis-semicarbazid oder Isophoron-bis-semicarbazid. Bevorzugt sind Hydrazin und nieder molekulare Verbindungen mit -CO-NH-$NH_2$-Gruppen mit Molekulargewichten von 32 bis 399. Besonders bevorzugt sind Hydrazinhydrat, β-Semicarbazidopropion-säurehydrazid und Alkenylenbissemicarbazide.

Als Komponente D können weiterhin besonders günstig auch offenkettige, monocyclische oder bicyclische Verbindungen, welche die Amidin- und/oder die Guanidingruppierung

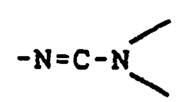

(I)

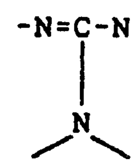

(II)

ein- oder mehrmals enthalten und welche zwei, ein oder keine bei Raumtemperatur gegenüber Isocyanatgruppen reaktive H-Atome besitzen, verwendet werden. Geeignete Amidine und Guanidine sind in DE-A-3 403 500 beschrieben.

Wie in DE-A-3 403 500 erläutert, reagieren derzeitige Verbindungen mit Isocyanaten in folgender Weise:

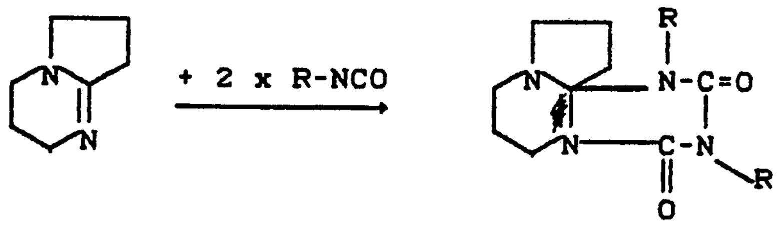

Beispiele für besonders bevorzugte monocylische Amidine sind:

1,2-Dimethyl-Δ-2-imidazolin,

1-Methyl-2-phenyl-Δ-2-imidazolin,

1(N)-Methyl-Δ2-imidazolin,

2-Benzylimino-N-methyl-caprolactam, 2-Butylimino-N-methyl-butyrolactam,
1,8-Diaza-bicyclo [5,3,0]-dec-7-en,
1,8-Diaza-biclo [5,4,0]-undec-7-en,
1,7-Diaza-bicyclo[4,4,0]-dec-6-en,
1,6-Diaza-bicyclo [3,4,0]-non-5-en
1,5-Diaza-bicyclo[4,3,0]-non-5-en,
1,14-Diaza-bicyclo[11,4,0]-heptadec-13-en,

1(N)-Methyl-Δ-2-tetrahydropyrimidin,

1-cyclohexyl-2-methyl-Δ2-tetrahydropyrimidin,

1-Cyclohexyl-Δ2-tetrahydropyrimidin,

1-Benzyl-2-butyl-Δ2-tetrahydropyrimidin,

1-Methyl-2-methyl-Δ2-tetrahydropyromidin,

1-Butyl-2-methyl-Δ2-tetrahydropyrimidin,

1- (2-Ethylhexyl)-2-methyl-Δ2-tetrahydropyrimidin,

1-Dodecyl-2-methyl-Δ2-tetrahydropyrimidin,

1-(1-Methylcyclohexyl)-2-methyl-Δ2-tetrahydropyrimidin,

1-(2-Methylhexyl)-2-methyl-Δ2-tetrahydropyrimidin,

1-(3,3,5-Trimethylcyclohexyl)-2-methyl-Δ2-tetra-hydropyrimidin.

Ganz besonders bevorzugte Amidine sind solche der Formel (III)

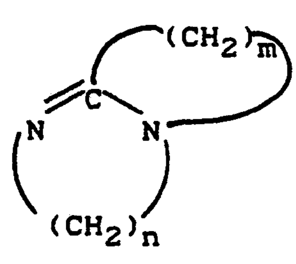

(III)

wobei

m 2, 3, 4, 5 oder 11 und
n 2, 3 oder 4 bedeutet.

Technisch besonders bevorzugt sind Tetramethylguanidin, Pentamethylguanidin und folgende cyclische Guanidinverbindungen

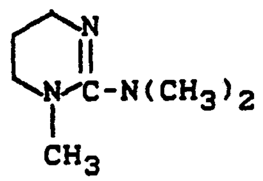

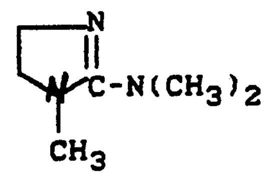

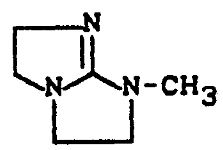

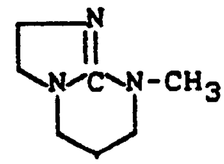

Anstelle der freien Amidinverbindungen können auch Säureadditionssalze der Amidine bzw. Guanidine eingesetzt werden, jedoch sind diese Salze weniger bevorzugt.

Bei Verwendung von offenkettigen, mono- oder bicylischen Amidinen oder Guanidinen bei der Umhüllung der festen Polyisocyanate A ist es von Vorteil, eine geringe Menge an Wasser, niedermolekularen Glykolen oder Aminen als zusätzliche Komponente D einzusetzen.

Die bei der Umhüllung verwendeten Amidine oder Guanidine zeigen oberhalb der Aufdickungstemperatur der Reaktivsysteme katalytische Wirksamkeit. Deshalb kann auf weitere Katalysatoren verzichtet werden, wie sie sonst für eine rasche Verfestigung von reaktionträgen H-Verbinodungen (z. B. Verbindungen mit sekundären OH-Endgruppen oder Wasser) benötigt würden.

Darüber hinaus können Amidine oder Guanidine in der Hitze weitere Reaktionen, wie z. B. die Uretdionringspaltung bei dimeren Polyisocyanaten, Trimerisierung, Allophanatisierung oder Biuretisierung bei den Polyisocyanaten A katalysieren. Diese Reaktionen können bei geeigneten Substraten zu einer beachtlichen Verbesserung der Haftwirkung führen.

Selbstverständlich können beliebige Kombinationen von Komponenten D verwendet werden, um z. B. nachteilige Nebeneffekte eines Amins oder Amidins durch entsprechende Vorteile anderer Amine oder Amidine auszugleichen (z. B. nieder- höhermolekulare Diamine in gemeinsamer Anwendung) oder um möglichst viele vorteilhafte Nebeneffekte zu vereinigen. In Frage kommen z. B. Kombinationen von schnell reagierenden Aminen wie z. B. Ethylendiamin mit durch sterische Hinderung verlangsamten Aminen oder von niedermolekularen Aminen mit hochmolekularen Aminen wie z. B. aliphatischen Aminopolyethern, oder von Polyaminen mit Hydrazinen oder Hydrazid-Derivaten. Beispielsweise verwendet man neben Hydrazin- oder Hydrazid-Derivaten oder Amidinen oder Guanidinen bis zu 50 %, an Polyaminen, bezogen auf die Gesamtmenge der eingesetzten Komponenten D.

Die Komponenten D werden in Mengen von mindestens 50, vorzugsweise 50 bis 100 Äquivalent-%, pro Äquivalent NCO des Polyisocyanats B, besonders bevorzugt in äquivalenten Mengen eingesetzt.

Die Umhüllung wird bei Temperaturen unter dem jeweiligen Schmelzpunkt des Polyisocyanats durchgeführt. Die Reaktionstemperatur liegt im allgemeinen unter 70° C, bevorzugt bei 0 bis 50° C.

Die Umhüllung des festen Isocyanats A erfolgt in der Regel innerhalb weniger Minuten, so daß auch eine kontinuierliche Arbeitsweise möglich ist.

Die Umhüllung der festen Polyisocyanate A wird in einem flüssigen Medium C, das kein Lösungsmittel für die festen Polyisocyanate A und insbesondere auch kein gutes Lösungsmittel für die zur Bedeckung dienenden Polyisocyanate B darstellt, durchgeführt. Die Suspensionsmittel C dienen gegebenen falls zur öligen Emulgierung oder gerade ausreichenden Lösung von B, insbesondere wenn B ein festes (weniger bevorzugt) Polyisocyanat ist. C darf nicht die Adsorption oder Abscheidung von B auf den festen Teilchen A verhindern. Geeignete Lösungsmittel, Weichmacher und Polyole wurden bereits aufgeführt.

Das flüssige Medium C ist bevorzugt ein höhermolekulares Polyol mit einem Molekulargewicht von 400 bis 6000. Vorzugsweise werden Polyole des Molekulargewichtsbereichs 400 bis 3000, besonders bevorzugt 1000 bis 3000, eingesetzt, gegebenenfalls unter Zusatz von niedermolekularen Polyolen.

Als flüssiges Medium C können auch Weichmacher verwendet werden, z. B. Adipate oder Phthalate, wie Dioctyl-, Diisododecyl-, Dibenzyl- oder Butylbenzylphthalat. Auch Kohlenwasserstoffe, wie sogenannte Butadienöle, oder Polyether mit höherem Molekulargewicht können als Suspensionsmittel C verwendet werden. Dabei wird im allgemeinen so verfahren, daß das feinpulverisierte feste Isocyanat A mit dem Polyisocyanat B bedeckt wird und mit einer Lösung von D im Weichmacher möglichst bei Raumtemperatur verrührt wird.

Die so erhaltenen umhüllten Polyisocyanate können in dieser Suspension weiterverarbeitet werden. Es ist aber auch möglich, die umhüllten Polyisocyanate aus der Suspension z. B. durch Filtration oder durch Abdampfen des Lösungsmittels zu isolieren und nachträglich in höhermolekularen Polyol- und Polyaminverbindungen wieder zu suspendieren. Diese Arbeitsweise ist nicht bevorzugt.

Die polymerumhüllten Polyisocyanate A können in Suspension, vorzugsweise in höhermolekularen Polyolen, bevorzugt aber in höhermolekularen Polyaminen mit aromatischen Aminogruppen, gegebenenfalls unter

Zusatz von niedermolekularen, aromatischen Polyaminen und/oder niedermolekularen Polyolen als Kettenverlangerungsmitteln, als Einkomponentenreaktivsysteme verwendet werden.

Man setzt dazu beispielsweise Suspensionen der bedeckten Polyisocyanate (A plus B) in Hexan oder Diisopropylether unter Rühren Polyolen oder höhermolekularen aromatischen Polyaminen zu, welche polymerbildende Verbindungen D gelöst enthalten.

Alternativ können die umhüllten Polyisocyanate A auch durch Umsetzung der Polyisocyanate B mit den Verbindungen D in wenig polaren Lösungsmitteln, Weichmachern oder gegebenenfalls Wasser hergestellt und die gebildeten umhüllten Polyisocyanate, z. B. durch Filtration, isoliert und erst anschließend in (höhermolekularen) Polyolen und/oder Polyaminen suspendiert werden.

Die erfindungsgemäß umhüllten Polyisocyanate A besitzen als Suspension in Polyolen oder höhermolekularen Polyaminen eine außerordentlich gute Lagerstabilität, auch bei erhöhten Temperaturen, selbst in Gegenwart hochwirksamer Polyurethankatalysatoren. Auch bei Verwendung der erfindungsgemäßen, umhüllten Polyisocyanate zusammen mit aromatischen Diaminen als Kettenverlängerer sind die Reaktivmischungen ausgezeichnet lagerstabil und zeigen als Gießsystem eine stark verlängerte Topfzeit, selbst wenn es sich um lösliche (flüssige) aromatische Polyamine handelt.

Die mit den umhüllten Polyisocyanaten gebildeten, langzeitlagerbeständigen, gut fließfähigen, gegebenenfalls leicht aufschmelzbaren, heterogenen Einkomponentensysteme lassen sich durch Zugabe von polaren Lösungsmitteln (z. B. Dimethylformamid) zur Aushärtung bringen; in manchen Fallen genügt auch schon eine Einwirkung von starken Scherkräften.

Bei Hitzehärtung können die erfindungsgemäßen Einkomponenten-Reaktivsysteme bereits in einem relativ niederen Temperaturbereich (oberhalb der Aufdickungstemperatur, bevozugt )⩾55°C, besonders bevorzugt 100 bis 135°C) zur Umsetzung gebracht werden, wobei hochwertige Polyurethankunststoffe hergestellt werden können.

Die bevorzugten höhermolekularen Polyole sind z. B. Polyoxyalkylen-polyole, z. B. Polyoxytetramethylenglykole oder Ethoxylierungs- und/oder Propoxylierungsprodukte von niedermolekularen Di- und Polyolen oder Di- und Polyaminen, z. B. propoxyliertes Trimethylolpropan, propoxyliertes Ethylendiamin oder lineare oder verzweigte Polypropylenglykolether, die anteilsweise in statistischer, blockartiger oder endständiger Form Ethylenoxid enthalten können und insgesamt Molekulargewichte von 400 bis 6000 aufweisen. In einer Ausführungform werden beispielsweise zwei- oder mehrfunktionelle höhermolekulare Polyole, gegebenenfalls unter Zusatz niedermolekularer Polyole als flüssiges Medium C zur Suspendierung der umhüllten Polyisocyanate verwendet, und können dann bei der Verwendung zum Aufbau von Polyurethanen direkt als Hydroxylgruppen tragende Reaktionsteilnehmer benutzt werden. Es können daher alle, üblicherweise für die Synthese von Polyurethanen eingesetzten höhermolekularen Verbindungen mit endständigen OH-Gruppen, wie Polyether, Polyacetale, Polythioether und auch Polyester verwendet werden, wie sie beispielsweise in der DE-A-2 920 501 aufgeführt sind. Die erfindungsgemäßen Einkomponenten-Polyurethansysteme können neben diesen (höhermolekularen) Polyolen auch entsprechende Mengen an niedermolekularen Polyolen, vorzugsweise Diolen, und/oder aromatischen Polyaminen, vorzugsweise Diaminen, enthalten (z. B. auch zusätzlich/nachträglich eingemischt), welche Molekulargewichte zwischen 60 und 399 aufweisen. Dem System werden besonders bevorzugt niedermolekulare aromatische Polyamine als Kettenverlängerungsmittel zugefügt.

Als höhermolekulare Polyhydroxylverbindungen sind zwei- oder höherwertige Polyhydroxylverbindungen mit 2 bis 8, vorzugsweise 2 bis 4, Hydroxylgruppen und einem Molekulargewicht von 400 bis 6000 geeignet. Dies sind mindestens zwei Hydroxylgruppen aufweisende Polyester, Polyether, Polythioether, Polyacetale, Polycarbonate, Polylactone oder Polyesteramide, sowie auch Polybutadienverbindungen oder ihre Mischungen, wie sie für die Herstellung von homogenen, gegebenenfalls zellförmigen oder schaumstoffartigen Polyurethanen an sich bekannt sind. Besonders bevorzugt sind Polyether und Polyester.

Die in Frage kommenden Polyether sind solche der an sich bekannten Art und werden z. B. durch Polymerisation von Tetrahydrofuran oder von Epoxiden wie Ethylenoxid, Propylenoxid, Butylenoxid, Styroloxid oder Epichlorhydrin bzw. durch Anlagerung dieser Epoxidverbindung, vorzugsweise von Ethylenoxid oder Propylenoxid, gegebenenfalls im Gemisch oder nacheinander, an Startkomponenten mit reaktionsfähigen Wasserstoffatomen, wie Wasser, mehrwertiger Alkohole, Ammoniak oder mehrwertige Amine oder Zucker, hergestellt.

Die in Frage kommenden, Hydroxylgruppen aufweisenden Polyester sind z. B. Umsetzungsprodukte von mehrwertigen, vorzugsweise zweiwertigen, und gegebenenfalls zusätzlich drei- und mehrwertigen Alkoholen mit mehrwertigen, vorzugsweise zweiwertigen, Polycarbonsäuren oder deren Anhydriden oder entsprechenden Polycarbonsäureestern von niedrigen Alkoholen.

Auch Polyester aus Lactonen, z. B. ε-Caprolacton, oder aus Hydroxycarbonsäuren, z. B. ω-Hydroxycapronsäure, sind einsetzbar, insbesondere wenn sie zusätzliche Komponenten, wie Diethylenglykol oder 1,4-Butandiol, enthalten, um ihre hohe Kristallinität zu mindern.

Als Polyacetale sind z. B. die aus Glykolen und Formaldehyd herstellbaren Verbindungen geeignet.

Als Hydroxylgruppen aufweisende Polycarbonate kommen solche der an sich bekannten Art in Betracht, die z. B. durch Umsetzung von Propandiol-1,3, Butandiol-1,4 und/oder Hexandiol-1,6, Di-, Tri- oder Tetraethylenglykol oder Thiodiglykol, mit Diarylcarbonaten, z. B. Diphenylcarbonat, oder Phosgen, hergestellt werden können.

Auch Hydroxylendgruppen aufweisende Polybutadiene sind erfindungsgemäß geeignet, da sie besonders

elastische und hydrolysenstabile Produkte ergeben. Es können gegebenenfalls auch Polyhydroxylverbindungen eingesetzt werden, in welchen hochmolekulare Polyaddukte bzw. Polykondensate oder Polymerisate in feindisperser oder auch gelöster Form enthalten sind.

Derartige Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen in situ in o.g. Hydroxylgruppen aufweisenden Verbindungen ablaufen läßt.

Auch durch Vinylpolymerisate modifizierte Polyhydroxylverbindungen, wie sie z. B. durch Polymerisation von Styrol und Acrylnitril in Gegenwart von Polyethern oder Polycarbonatpolyolen erhalten werden, sind für das erfindungsgemäße Verfahren geeignet.

Weitere Vertreter der genannten zu verwendenden Verbindungen sind z. B. in High Polymers, Vol. XVI, "Polyurethanes, Chemistry and Technology", verfaßt von Saunders-Frisch, Interscience Publishers, New York, London, Band I, 1962, Seiten 32 bis 42 und Seiten 44 bis 54 und Band II, 1964, Seiten 5 bis 6 und 198 bis 199, sowie im Kunststoff-Handbuch, Band VII, Vieweg-Höchtlen, Carl-Hanser-Verlag, München, 1966, z. B. auf den Seiten 45 bis 71, sowie in der DE-A-2 854 384 ausführlich beschrieben.

Es ist selbstverständlich möglich, Mischungen der o.g. Polyhydroxylverbindungen einzusetzen.

Wie schon erwähnt, können die erfindungsgemäßen Einkomponenten-Reaktivsysteme auch flüssige oder niedrigschmelzende (50°C) nieder- und/oder höhermolekulare aromatische Polyamine und/oder höhermolekulare aliphatische Polyamine enthalten.

Als höhermolekulare Polyaminoverbindungen mit aromatischen Aminogruppen mit einem Molekulargewichtsbereich von 400 bis 6000 werden insbesondere solche Polyaminoverbindungen eingesetzt, wie sie durch Hydrolyse von entsprechenden NCO-Prepolymeren auf der Basis von höhermolekularen Polyhydroxylverbindungen und überschüssigen aromatischen Diisocyanaten durch (vorzugsweise basische) Hydrolyse hergestellt werden können. Beispiele für dieses Verfahren werden in DE-A-2 948 419, DE-A-3 039 600, DE-A-3 112 118, EP-A-61 627, EP-A-71 132, EP-A-71 139 und EP-A-97 869 angegeben. In der erstgenannten Druckschrift werden auch weitere Verfahren des Standes der Technik zur Herstellung von geeigneten aromatischen Aminoverbindungen höhermolekularer Struktur genannt. Es handelt sich bei dem Verfahren nach DE-A-2 948 419 und den anderen zitierten Druckschriften vorzugsweise um Polyether-Polyamine, aber auch Polyester-, Polyacetal-, Polythioether- oder Polycaprolacton-Polyamine, vorzugsweise 2- der 3-funktionelle Polyamine, welche Urethangruppen (aus der Reaktion der entsprechenden höhermolekularen Polyhydroxylverbindungen mit den überschüssigen Polyisocyanaten) enthalten und die Aminogruppen am Rest des (ehemaligen) Polyisocyanats tragen. Die aromatischen, höhermolekularen Polyamine können jedoch auch nach anderen Verfahren, z. B. durch Umsetzung von NCO-Prepolymeren mit überschüssigen Mengen an Hydrazin, Aminophenylethylamin, oder anderen Diaminen entsprechend DE-B-1 694 152 hergestellt werden; eine andere Synthesemöglichkeit beschreibt die FR-A-1 415 317 durch Überführung der NCO-Prepolymere mit Ameisensäure in die N-Formylderivate und deren Verseifung. Auch die Umsetzung von NCO-Prepolymeren mit Sulfaminsäure gemäß DE-B-1 155 907 führt zu höhermolekularen Polyaminen. Neben an aromatische Reste gebundenen Aminogruppen (aus aromatischen Polyisocyanaten) lassen sich auch (über aliphatische Polyisocyanate) an aliphatische Reste gebundene, höhermolekulare Polyaminoverbindungen verwenden. Diese höhermolekularen, aliphatischen Polyamine können auch als Reaktionskomponente D Verwendung finden, wenn die Umhüllungsreaktion bei niedrigen Temperaturen, z. B. Raumtemperatur, durchgeführt wird. Wird die Temperatur entsprechend gesteigert, z. B. auf 120°C, so tritt eine Durchreaktion aller aliphatischen Aminogruppen mit den Isocyanatgruppen ein. Hierbei können direkt Elastomere erhalten werden, wenn das Verhältnis zwischen NCO-Gruppen und NCO-reaktiven Gruppen (OH- und/oder $NH_2$-Gruppen) so gewählt wird, daß ein polymeres Produkt entsteht.

Den genannten Suspensionmittel C können gegebenenfalls Lösungsmittel unpolarer oder wenig polarer Art zugemischt werden, z. B. aliphatische, cycloaliphatische oder aromatische Kohlenwasserstoffe, Halogenkohlenwasserstoffe, Ether, Ketone oder Ester, vorzugsweise mit Siedepunkten unter 146°C. Hierdurch läßt sich gegebenenfalls eine Reaktion in einem niedrigerviskosen Medium erreichen; die Lösungsmittel werden zweckmäßig anschließend wieder entfernt, z. B. durch Abziehen im Vakuum.

Die geschilderten Umhüllungsreaktionen führen zu einer Suspension von polymerumhüllten, stabilisierten Polyisocyanaten A in dem flüssigen Medium C.

Die Suspensionen enthalten in der Regel mindestens 3 Gew. -%., bevorzugt mindestens 5 Gew.-%, und in den meisten Fällen mindestens 7,5 Gew.-% feste umhüllte Polyisocyanate; die Feststoffgehalte liegen zumeist unter 70 Gew-%, vorzugsweise unter 50 Gew.-% und in den meisten Fällen unter 40 Gew.-%.

Sind die umhüllten Polyisocyanate in einem solchen Medium, z. B. höhermolekularen Polyolen oder Polyaminen, suspendiert, welches für die weiteren Reaktionen zu Polyurethanen geeignet ist, so kann diese Suspension direkt weiterverwendet werden. Gegebenenfalls - wenn auch weniger bevorzugt - werden jedoch die umhüllten Polyisocyanate aus der Suspension, vor allem bei Anwendung von Wasser oder sehr viel Weichmacher und/oder Lösungsmittel, z. B. durch Filtration isoliert und als Pulver den anderen zum Aufbau des Polyurethans dienenden Reaktionskomponenten (höhermolekularen Polyolen und/oder Polyaminen, sowie gegebenenfalss niedermolekularen Kettenverlängerungsmitteln) zugesetzt. Leichflüchtige Lösungsmittel C können aus den Suspensionen (vorzugsweise unter Anlegen von Vakuum) abdestilliert werden.

Besonders wichtig für die Praxis sind solche lagerstabilen Suspensionen umhüllter Polyisocyanate in höhermolekularen Polyaminen (gegebenenfalls unter Zusatz höhermolekularer Polyole und/oder

Kettenverlängerungsmittel, z. B. niedermolekularen Polyaminen und niedermolekularen Polyolen), wie sie direkt als Einkomponentensystem bzw. für die Formulierung von Einkomponentensystemen verwendet werden können. Bevorzugt ist eine Verwendung der Komponenten A - D in solchen Mengen- und Äquivalentverhältnissen, daß sie direkt einer Rezeptur von Einkomponenten-Reaktivsystemen entsprechen.

Wie bereits erwähnt, werden die erfindungsgemäßen, langzeitlagerbeständigen Einkomponenten-Reaktivsysteme vorzugsweise unter Mitverwendung von niedermolekularen Kettenverlängerern oder Vernetzern hergestellt. Bei diesen niedermolekularen Kettenverlangerern oder Vernetzern handelt es sich um zwei- oder mehrfunktionelle Verbindungen, welche an aliphatische und/oder cycloaliphatische Gruppen gebundene Hydroxylgruppen und/oder an aromatische Ringe, einschließlich heterocyclische Ringe mit aromatischem Charakter, gebundene $NH_2$-Gruppen und Molekulargewichte zwischen 62 und 399 aufweisen. Bevorzugt sind dabei niedermolekulare Diole mit an aliphatische oder cycloaliphatische Gruppen gebundenen Hydroxylgruppen, sowie aromatische Diamine des genannten Molekulargewichtsbereichs.

Diese Verbindungen weisen in der Regel 2 bis 8, vorzugsweise 2 bis 4, besonders bevorzugt jedoch 2, gegenüber Isocyanaten reaktionsfähige Wasserstoffatome wie Hydroxyl- und/oder Aminogruppen auf. Es können selbstverständlich auch Mischungen von verschiedenen Verbindungen dieser Art verwendet werden. Als Beispiele für derartige Verbindungen seien genannt: Ethylenglykol, Trimethylenglykol, Butandiol-2,3 und/oder -1,4,Hexandiol-1,6, Neopentylglykol, 1,4-Bis-hydroxyethyl-cyclohexan, 1,4-Dihydroxycyclohexan, Terephthalsäure-bis(β-hydroxyethyl)-ester, 1,4,3,6-Dianhydrohexite, 1,4-Monoanhydrotetrite, sowie weniger bevorzugt Diole mit sekundären Hydroxylgruppen, z. B. Propylenglykol, Butandiol-2,3, oder Pentandiol-2,5. Als mehrwertige Verbindungen seien genannt:

Methylolethan, Hexantriol-1,2,6, Glycerin, Pentaerythrit, Chinit, Mannit, Sorbit, Rizinusöl, sowie Di-, Tri- und Tetraethylen-, -propylen-, und -butylen-glykole, ferner Bis-(2-hydroxyethyl)-hydrochinon, Bis-(2-hydroxyethyl)-resorcin, Formose oder Formit. Ferner sind geeignet tertiäraminhaltige Di- oder Polyole, z. B. N-Methyldiethanolamin, Triethanolamin oder N,N'-Bis-hydroxyethylpiperazin.

Bevorzugt werden jedoch anstelle von niedermolekularen Polyolen niedermolekulare aromatische Diamine eingesetzt. Unter aromatischen Polyaminen sollen auch solche Amine verstanden werden, welche die Amionogruppe an heterocyclische Reste mit aromatischem Charakter gebunden enthalten. Als aromatische Polyamine sind z. B. geeignet: p-Phenylendiamin, 2,4/2,6-Toluylendiamine, Diphenylmethan-4,4'- und/oder -2,4' und/oder -2,2'-diamine, 3,3'-Dichlor-4,4'-diaminodiphenylmethan, 3-($C_1$-$C_8$)-Alkyl-4,4'-diaminodiphenylmethane, die 3,3'-Di-($C_1$-$C_4$)-4,4'-diaminodiphenylmethane sowie die 3,3', 5,5'-Tetra-($C_1$-$C_4$)-alkyl-4,4'-diphenylmethane, die 4,4'-Diaminodiphenyl-sulfide, -sulfoxide oder -sulfone, Ethergruppen aufweisende Diamine gemäß DE-A-1 770 525 und 1 809 172 (US-A-3 654 364 und 3 736 295), gegebenenfalls in 5-Stellung substituierte 2-Halogen-1,3-phenylendiamine (DE-A-2 001 772, 2 025 896 und 2 065 869), Bisanthranilsäureester (DE-A-2 040 644 und 2 160 590), 2,4-Diaminobenzoesäureester nach DE-A-2 025 900, sowie durch eine oder zwei ($C_1$-$C_4$)-Alkylgruppen substituierte Toluylendiamine. Besonders bevorzugt sind 3,5-Diethyl-2,4- und/oder - 2,6-diaminotoluol (besonders ihre technischen (80/20) - oder (65/35)-Isomerengemische), unsymmetrisch tetraalkylsubstituierte Diaminodiphenylmethane, z. B. 3,5-Diethyl-3',5'-diisopropyl-4,4-diaminodiphenylmethan und ihre Isomerengemische entsprechend DE-A-2 902 090, 4,4'-Diaminobenzanilid, sowie 3,5-Diaminobenzoesäure-($C_1$-$C_4$)-alkylester, 4,4'- und/oder 2,4'-Diaminodiphenylmethan, sowie Naphthylen-1,5-diamin.

Weitere Beispiele für Kettenverlängerungsmittel sind Adipinsäure-bis-(2-hydroxyethyl)-ester, Terephthalsäure-bis-(2-hydroxyethyl)-ester, Diol-urethane, Diol-harnstoffe oder Polyole, welche Sulfonat- und/oder Phosphonatgruppen enthalten, z. B. 1,6-Hexamethylen-bis-(2-hydroxyethylurethan), 4,4'-Diphenylmethan-bis-(2-hydroxyethylharnstoff) oder das Addukt von Na-Bisulfit an Butendiol-1,4, bzw. dessen Alkoxylierungsprodukte. Weitere niedermolekulare Verbindungen werden ausführlich in der DE-A-2 854 384 beschrieben.

Ferner können in üblicher Weise gegebenenfalls gegenüber Isocyanaten monofunktionelle Verbindungen in Anteilen von 0,01 bis 10 Gew.-% als sogenannte Kettenabbrecher mitverwendet werden. Derartige monofunktionelle Verbindungen sind z. B. Monoamine, wie Butyl- oder Dibutylamin, Stearylamin, Pyrrolidin, Anilin oder Tolylamin, Butanol, 2-Ethylhexanol, Cyclohexanol oder Ethylenglykolmonoethylether.

Als Katalysatoren in den erfindungsgemäßen, langzeitlagerstabilen Einkomponentensystemen sind die üblichen Polyurethankatalysatoren geeignet; besonders organische Blei- und/oder Zinnverbindungen, gegebenenfalls unter Mitverwendung weiterer, üblicher Polyurethankatalysatoren, insbesondere von tert.-Amin-haltigen Katalysatoren.

Von den Bleiverbindungen werden Verbindungen aus folgender Gruppe bevorzugt:

a) organische Salze des zweiwertigen Bleis mit Carbonsäuren,
b) Dithiocarbamate des zweiwertigen Bleis.

Als organische Zinn-verbindungen kommen Zinn-(II)-salze von Carbonsäuren wie Zinn-acetat, Zinn-octoat, Zinnethylhexanoat und Zinn-laurat, und die Zinn-(IV)-Verbindungen, z. B. Dibutylzinnoxid, Dibutylzinndichlorid, Dibutylzinndiacetat, Dibutylzinndilaurat, Dibutylzinnmaleat oder Dibutylzinndiacetat in Betracht. Bevorzugt verwendet werden bei den Zinnkatalysatoren jedoch schwefelhaltige Zinnverbindungen.

Von Interesse sind auch Kombinationen der organischen Metallverbindungen mit Amidinen, Aminopyridinen, Hydrazinopyridinen (DE-A-2 434 185, 2 601 082 und 2 603 834), oder 1,4-Diaza-bicyclo-2.2.2.-octan und/oder tert.-

Amin-Katalysatoren, wie sie in der Polyurethanchemie üblicherweise verwendet werden.

Die Bleikatalysatoren sind dabei von besonderer Wirksamkeit und Effektivität, wenn das Reaktivsystem Polyetherpolyole mit sekundären Hydroxylgruppen, z. B. Polypropylenoxidglykole enthält.

Bei Einsatz von Uretdiondiisocyanaten A kann insbesondere bei Bleikatalysatoren auch eine zusätzliche Vernetzung infolge Spaltung des Uretdionringes erfolgen, wobei verzweigende Allophanatgruppen oder bei vollständiger Spaltung des Uretdionrings zusätzlich Urethangruppen gebildet werden.

Bei Einsatz von (Polyester)polyolen mit im wesentlichen primären Hydroxylgruppen sind dagegen die Zinnverbindungen, besonders die Zinn/Schwefel-Katalysatoren, von besonderer Wirksamkeit. Bei aromatischen $NH_2$-gruppenhaltigen Polyethern kann zumeist auf eine Katalyse völlig verzichtet werden. Die Katalysatoren werden in der Regel in einer Menge von 0,001 bis 5 Gew.-%, vorzugsweise von 0,01 bis 2 Gew.-%, bezogen auf A + B, eingesetzt.

Als in den erfindungsgemäßen Einkomponenten-Reaktivsystemen gegebenenfalls einzusetzende Hilfs- und Zusatzstoffe seien genannt: Farbstoffe oder Pigmente, Füllstoffe wie Silicagel, Gips, Talkum, Aktivkohle, Metallpulver, UV-Absorptionsmittel oder Stabilisatoren wie phenolische Antioxidantien, Lichtschutzmittel, Treibmittel, oberflächenaktive Zusatzstoffe wie Emulgatoren oder Schaumstabilisatoren, gegebenenfalls Zellregler, Antiblockmittel, Silikone, Flammschutzmittel und fungistatisch und/oder bakteriostatisch wirkende Substanzen.

Als Füllstoffe sind z. B. Fasermaterialien, d.h. alle an sich bekannten anorganischen und/oder organischen, faserförmigen Verstärkungsmaterialien einsetzbar.

Die einzubauende Füllstoffmenge hängt von der gewünschten Verbesserung der mechanischen Eigenschaften ab, im allgemeinen werden 5 bis 60 Gew.-% Fasermaterial angewendet.

Bei der Verwendung der erfindungsgemäßen umhüllten Polyisocyanate zur Herstellung von Polyurethanen (besonders in Form von Einkomponenten-Reaktivsystemen) beträgt das $NCO/(NH_2 + OH)$ Verhältnis (NCO aus umhülltem Polyisocyanat A und gegebenenfalls verwendetem weiterem, freien Polyisocyanat; Amino- und OH-Gruppen aus höhermolekularen Polyolen und/oder Polyaminen und/oder Kettenverlängerungsmitteln) bei der Polyurethanbildung 0,5 : 1 bis 1,5 : 1, bevorzugt 0,8 : 1 bis 1,5 : 1, besonders bevorzugt 0,95 : 1 bis 1,2 : 1 (Angaben in Äquivalenten).

Pro $(OH + NH_2)$-Äquivalent an höhermolekularen Polyolen und/oder Polyaminen werden dabei insbesondere 0,3 bis 10, bevorzugt 0,5 bis 8 und besonders bevorzugt 0,75 bis Äquivalente an $(OH + NH_2)$-Äquivalenten an Kettenverlängerungsmitteln, d.h. niedermolekularen Polyolen oder niedermolekularen Polyaminen, in den Polyurethan-Reaktiv-Mischungen eingesetzt.

Uretdionringe enthaltende (dimere) Diisocyanate A können im allgemeinen als Diisocyanate betrachtet werden, so daß bei Berechnung der obigen Äquivalentverhältnisse nur die freien NCO-Gruppen berücksichtigt werden. Unter bestimmten Versuchsbedingungen (Verwendung von Bleikatalysatoren, höhere Verarbeitungstemperatur, z. B. $>140°C$) greift der Uretdionring jedoch mit in die Reaktion ein (z. B. über die Bildung zusätzlicher Verknüpfungsstellen über Allophanat- oder Biuretgruppen), so daß die latenten NCO-Gruppen des Uretdionrings in diesem Fall bei der Berechnung mitberücksichtigt werden müssen.

Je nach Viskosiät bzw. Schmelzverhalten der Ausgangskomponenten erhält man bei Raumtemperatur gut gießbare, rakelfähige oder auch feste, leicht aufschmelzbare Einkomponenten-PU-Reaktivmischungen. Diese Reaktivmischungen stellen dabei eine heterogene Suspension der festen, umhüllten Polyisocyanate in den Polyol- und/oder Polyaminkomponenten dar. Die Hitzevernetzung dieser Mischung erfolgt zumeist erst nach Zugabe geeigneter Katalysatoren. Ohne diese Katalysatoren werden insbesondere bei Verwendung von höhermolekularen Polyolen oder Polyol-Kettenverlängerungmitteln keine befriedigenden Eigenschaften oder Polyurethanformkörper erhalten. Bei alleiniger Verwendung der gegenüber NCO-Gruppen deutlich reaktiveren aromatischen Polyaminverbindungen kann jedoch auf einen Zusatz von Katalysatoren verzichtet werden.

Ein weiteres Merkmal der erfindungsgemäßen Einkomponenten-Reaktivsysteme ist, daß sie nach Erreichen einer, von Menge und Art der Umhüllung abhängigen, Temperatur innerhalb weniger Minuten vernetzen. Das bedeutet, daß einerseits unterhalb dieser Temperatur ("Aufdickungstemperatur") mit der noch unvernetzten Reaktivmischung ein völliges Ausfüllen der heißen Werkzeugform auch bei langen Fließwegen möglich ist, andererseits aber die folgende rasche Verfestigung der Gießansätze nach Temperaturerhöhung schnelle Entformungszyklen gestattet.

Besonders günstig ist die selbst bei höheren Lagertemperaturen (z. B. bis 60°C) sehr lange Lagerzeit der erfindungsgemäßen Reaktivsysteme.

Mit den erfindungsgemäßen Polyisocyanatsuspensionen sind die Verwendungsmöglichkeiten von Einkomponentensystemen deutlich verbreitert; es können auch flüssige höhermolekulare Polyamine und Polyole eingesetzt werden und es sind auch nicht nur ausgewählte Kettenverlängerungsmittel (z. B. hochschmelzende Kettenverlängerungsmittel) einsetzbar. Ein wesenlicher Vorteil der erfindungsgemäßen Einkomponentensysteme besteht darin, daß man in diesen Systemen auch aromatische Diamine, wie z. B. 4,4'-Diaminodiphenylmethan, 2,4- oder 2,6-Diaminotoluol, 3,5-Diethyl-2,4/2,6-(65/35)-diaminotoluol, 1,5-Diaminonaphthalin oder 3,5-Diethyl-3',5'-diisopropyl-4,4'-diaminodiphenylmethan als Kettenverlängerungsmittel verwenden kann, ohne die bei Einkomponentensystemen gewünschte lange Verarbeitungszeit einzubüßen. Werden dagegen diese Diamine in einer der bisher üblichen Verfahrenstechniken mit NCO-Prepolymeren umgesetzt, so resultieren extrem kurze Gießzeiten, so daß ein einwandfreier Verlauf dieser Ansätze im Werkzeugteil nicht möglich ist.

Durch die Verwendung von höhermolekularen Polyaminen in dem Einkomponentensystem lassen sich

Polyurethanharnstoffe mit deutlich günstigeren Eigenschaften wie z. B. höheren Festigkeiten, höheren Spannungswerten, höherer Härte und höheren Erweichungsbereichen, erzielen als mit höhermolekularen Polyolen alleine.

Die erfindungsgemäßen, gegebenenfalls katalysatorhaltigen Einkomponentensysteme werden vorzugsweise durch Hitzestoß verfestigt. Bei Raumtemperatur oder wenig erhöhter Temperatur tritt auch in Gegenwart stark wirksamer Katalysatoren überraschenderweise keine Vernetzungsreaktion ein, so daß man auch katalysatorhaltige Mischungen als langzeitlagerstabile Einkomponentensysteme bezeichnen kann.

Die Verarbeitung der erfindungsgemäßen Einkomponentensysteme richtet sich nach deren Beschaffenheit. Flüssige, bei Raumtemperatur gießbare Systeme lassen sich im Gießprozeß verarbeiten, gegebenenfalls werden sie vor der Verarbeitung kurz erwärmt, z. B. auf 50 - 70°C. Die Verarbeitung kann auch im Schleuderguß erfolgen; Hohlkörper können durch Einbringung der Reaktivmasse in beheizte Formen unter Verteilung an der Oberfläche durch entsprechende Rotationsbewegungen hergestellt werden.

Auch nach dem slush-molding-Process können beheizte Formen mit der Reaktivmasse gefüllt werden, wobei nach einer gewissen Ausheizzeit/Reaktion an der beheizten Formoberfläche überschüssige, noch nicht reagierte Reaktionsmasse aus den Formen wieder ausgegossen wird.

Bei Mitverwendung von Treibmitteln können zellige Polyurethane hergestellt werden, die gegebenenfalls eine integrale Dichtestruktur aufweisen.

Nicht mehr gießbare, aber noch verlaufende Systeme können z. B. mit einer Rakel auf gewünschte Unterlagen, z. B. textile Unterlagen, z. B. Vliese, Gewirke und Gewebe, (Spalt)leder, Matrizen (z. B. Velourleder-Silikon-Matrizen), oder Zwischenträger (z. B. Trennpapiere) unter Bildung von Beschichtungen oder Zurichtungen, aufgebracht und anschließend durch Hitzestoß verfestigt werden.

Plastische Systeme (Pasten) können unter Druck- und Formgebung in der Hitze verpreßt werden, wobei bei 120°C bereits 5 bis 15 Minuten zur Verfestigung ausreichend sind.

Auch durch Tauchverfahren können Oberflächenbeschichtungen, Abdruckformen oder Formkörper hergestellt werden, indem man die beheizten, zu beschichtenden Formen in die Reaktivmasse eintaucht.

Die Reaktivmasse kann auch durch Schlitze oder Düsen in heiße Medien Heißluft oder heiße Flüssigkeiten) ausgepreßt und dadurch verfestigt werden.

Die Reaktivmasse kann in beheizten Extrudern teilweise oder weitgehend zum Polyurethan reagiert werden und in dieser Form durch Schlitze oder Düsen extrudiert und gegebenenfalls in heißen Medien vollständig ausreagiert werden; oder in eine heiße Form eingebracht werden, woraus sie nach kurzer Zeit entformt werden kann. Auch nach den Reaktionsspritzgußverfahren (reaction-injection-molding - RIM -) kann die Reaktivmasse verarbeitet werden.

Feste Systeme, insbesondere auf Basis höherschmelzender Polyole (45 bis 65°C) werden entweder unter Druck und Formgebung (Spritzguß) oder etwa bei oder oberhalb der Schmelztemperatur des Polyols verarbeitet. Man kann so verfahren, daß man die vorher hergestellten Einkomponentensysteme in Form fester Granulate in eine über den Schmelzpunkt des Polyols aufgewärmte Form (im allgemeinen unterhalb 70°C) einbringt. Nach dem Aufschmelzen der Granulate und der damit gegebenen Werkzeugfüllung wird die Form auf 100 bis 120°C erwärmt und der Inhalt verfestigt.

Die Verfestigungstemperatur der erfindungsgemäßen Einkomponentensysteme ist von Menge und chemischer Konstitution der Polyisocyanate B und der Komponenten D, die zur Umhüllung der Polyisocyanate A verwendet wurden, stark abhängig. Mit zunehmender Verfestigungstemperatur nimmt die aufzuwendende Verfestigungszeit zur Bildung der Polyurethane ab. Die Ausheizdauer kann je nach Temperatur von weniger als 1 Minute bis zu mehreren Stunden betragen. Manchmal ist es vorteilhaft, die Kunststoffe nach Entformen noch einige Zeit bei 100°C zu tempern, um eine vollständige Durchhärtung zu gewährleisten. Die erfindungsgemäßen Polyisocyanat-Reaktivsysteme gestatten insbesondere auch die Herstellung großvolumiger Formteile, ohne daß sich die Aufdickungstemperatur des Systems ändert.

Eine Aushärtung der Einkomponenten-Reaktivsysteme kann jedoch auch durch Zugabe von vorzugsweise hochpolaren Lösungsmitteln wie Dimethylformamid, N-Methylpyrrolidon, oder mäßig polaren Lösungsmitteln wie Propylencarbonat, Dioxan oder Glykolmonomethyletheracetat erfolgen. Je nach Menge dieser Lösungsmittel kann die Schutzhülle um die Polyisocyanate A in den Einkomponentensystemen zum Teil oder völlig entfernt werden. Die Gießzeit (Topfzeit) solcher Ansätze kann daher durch die Menge solcher Lösungsmittelzusätze gesteuert werden. Bei geringen Mengen erhält man Systeme mit mehrtägiger Topfzeit bei Raumtemperatur, während bei höheren Dosierungen bereits nach 10 bis 15 Minuten schnelle oder gar schlagartige Verfestigung eintritt. Die Zusatzmenge solcher Lösungsmittel ist auch hier von der Menge und Art der Hülle (Güte der Polyadduktaut an der Isocyanatoberfläche) abhängig und wird nach praktischen Vorversuchen für die jeweiligen Systeme festgelegt. Der technische Vorteil solcher Reaktionsmischungen liegt darin, daß diese Systeme auch ohne Zufuhr von Wärme verfestigen. Selbstverständlich kann man auch durch geeignete Dosierung der Lösungsmittel die thermische Verfestigungszeit der Einkomponentensysteme erniedrigen, wobei noch eine ausreichende Lagerstabilität gegeben sein kann.

Weiterhin kann eine Verfestigung der erfindungsgemäßen Einkomponentensysteme auch durch Anwendung hoher Scherkräfte herbeigeführt werden, so z. B. in hochtourigen Rührwerken. Die dabei bei kurzzeitigem Rühren auftretende Wärmetönung bewirkt in der Regel keine Erwärmung bis zur Aufdickungstemperatur der Einkomponentensysteme, so daß lediglich durch mechanische Beanspruchung während des Rührprozesses die auf der Isocyanatteilchenoberfläche befindliche Polymerhaut zerstört wird.

Bevorzugte Verwendung finden solche PU-Reaktivsysteme, welche höhermolekulare Polyamine und/oder

Kettenver längerungsmittel - vorzugsweise niedermolekulare aromatische Polyamine - als Komponenten enthalten und somit hochwertige Elastomere, Beschichtungen, zellige Elastomere und Formteile mit gegebenenfalls einer Dichteverteilung mit zelligem Innenkern und dichterer Außenhaut ergeben.

Bestimmung der "Aufdickungstemperatur" (ADT) von Einkomponenten-Reaktivsystemen.

Es werden Proben der flüssigen oder pastenartigen Suspension von umhüllten Polyisocyanaten A in höher- und/oder niedermolekularen Polyolen und/oder Polyaminen (als Einkomponenten-Reaktivsysteme) in einer ca. 1 cm breiten Spur auf einer Koflerbank aufgestrichen und der Beginn der Verfestigung des Systems zu einem Polyurethan nach einer Wartezeit von 15 Minuten bestimmt und die zugehörige "Aufdickungstemperatur" abgelesen.

(Für relativ reaktive Systeme, wie sie z. B. für RIM-Verfahren benutzt werden sollen, kann auch der Wert nach 5 oder 10 Minuten bestimmt werden).

Es wurden folgende Abkürzungen in der Beschreibung bzw. in den Beispielen verwendet:

| | |
|---|---|
| TDI | = 2,4-Toluylendiisocyanat |
| TT | = feinteiliges, festes Dimeres des 2,4-Toluylendiisocyanats |
| N | = biuretisiertes Hexan-1,6-diisocyanat (ca. 21,7 % NCO in der Festsubstanz); Desmodur®N der Bayer AG, Leverkusen |
| HDI-Trim. | trimerisiertes Hexan-1,6-diisocyanat |
| HDI | = Hexan-1,6-diisocyanat |
| NDI | = Naphthalin-1,5-diisocyanat |
| MDL | = Diphenylmethan-4,4'-diisocyanat |
| Dim-MDI | = dimeres/oligomeres MDI (Uretdiongruppenhaltig) |
| TDIH | = 4,4'-Dimethyl-3,3-diisocyanatodiphenylharnstoff |
| IPDI | = Isophorondiisocyanat |
| IPDI-Trim | Trimerisat von IPDI |
| DMDAD | = 3,3'-Dimethyl-4,4'-diamino-dicyclohexylmethan; Laromin®C260 der BASF, Ludwigshafen |
| DIP | = Diisopropylether |
| AAPE | = aromatischer Aminopolyether, NH-Zahl 48,4, hergestellt nach dem Verfahren der DE-A-3 039 600 durch alkalische Verseifung eines NCO-Prepolymers aus einem linearen Polyoxy propylendiol (Molgewicht 2000) und Toluylen-2,4-diisocyanat im Molverhältnis 1 : 2. |

**Beispiel 1**

**I. Bedeckung von festen Di- oder Polyisocyanaten mit flüssigen Di- oder Polyisocyanaten**

1.1 Bedeckung von TT mit N

1.1.1 Verfahren zur Bedeckung

Zu einer Suspension von 250 g TT in 850 g Hexan wird eine Lösung von 62,5 g N, mit einem NCO-Gehalt von 21,3 Gew.-%, in 30 g Toluol innerhalb von 30 Minuten unter starkem Rühren bei Raumtemperatur zugetropft. Nach einer Stunde wird das feste Endprodukt abgesaugt und getrocknet (312 g Ausbeute). Im Filtrat ist praktisch kein gelöstes Isocyanat mehr nachweisbar.

1.1.2 Charakterisierung des bedeckten Polyisocyanats nach Verfahren 1.1.1

Man erhält ein rieselfähiges, weißes Pulver, das im Kern das feste TT und an der Oberfläche das (an sich öligharzartige) biuretisierte Polyisocyanat N in einem Anteil von 20 %, adsorbiert enthält.

Vergleichsversuch zu 1.1.1 (in Toluol als alleinigem Lösungsmittel).

Zu einer Suspension von 250 g TT in 850 g Toluol werden unter Rühren 62,5 g N in 30 g Toluol zugesetzt. Nach einer Stunde wird das feste Polyisocyanat abgesaugt und mit wenig Petrolether gewaschen. Man erhält praktisch nur die eingesetzte Menge an TT zurück (245 g effektiv) und im Filtrat ist das Polyisocyanat N in der erwarteten Konzentration über NCO-Titration bestimmbar. Es erfolgt also keine wirksame "Bedeckung" von TT mit N in Toluol als alleinigem Lösungsmittel. Toluol stellt offensichtlich ein zu gutes Lösungsmittel für das Polyisocyanat N dar, das sich somit nicht (hinreichend) auf dem TT abscheidet, sondern in Toluol gelöst bleibt.

1.2 Bedeckung von NDI mit N

1.2.1 Verfahren zur Bedeckung:

Zu einer Suspension von 20 g NDI, NCO-Gehalt = 39,4 %, in 50 g Hexan wird eine Lösung von 5 g

Polyisocyanat N in 5 g Toluol unter starkem Rühren bei Raumtemperatur zugesetzt. Nach kurzer Zeit wird das feste Produkt abgesaugt und getrocknet. Man erhält eine Ausbeute von 24,5 g. Im Filtrat ist praktisch kein NCO nachweisbar.

1.2.2 Charakterisierung des bedeckten Polyisocyanats nach Verfahren 1.2.1

Das bedeckte Isocyanat stellt ein rieselfähiges Pulver mit einem NCO-Gehalt von 35,6 % (berechnet 35,9 % auf Mischung NDI/N) dar und besitzt einen Erweichungspunkt von 120 bis 121° C.

1.3 Bedeckung von MDL mit IPDI-Trim

1.3.1 Verfahren zur Bedeckung

Zu einer Suspension von 20 g festem, kristallisiertem MDI in 50 g Hexan (im Eisbad unter 10° C gekühlt) wird eine Lösung von 7,5 g IPDI-Trim in 5 g Toluol zugetropft. Nach 30 minütigem Rühren wird abgesaugt und im Wasserstrahlvakuum bei Temperaturen von 20° C getrocknet. Das Filtrat der Lösungsmittel ist praktisch frei von Polyisocyanaten.

1.3.2 Charakterisierung des bedeckten Polyisocyanats:

Das Produkt stellt ein rieselfähiges, weißes Pulver mit einem NCO-Gehalt von 28 Gew.-% (berechnet 28,4 Gew.-%) dar und besitzt einen Schmelzpunkt von 39 bis 40°.

**II. Desaktivierungsreaktion der mit flüssigen Polyisocyanaten bedeckten festen Addukte durch Polymerumhüllung**

1.4.1

Zu einer Suspension von 25 g des bedeckten Polyisocyanats (Adduktes) gemäß Beispiel 1.1 in 150 g Hexan werden 1,5 g 4,4'-Diamino-3,3'-dimethyl-dicyclohexylmethan (DMDAD) zugesetzt. Das Äquivalentverhältnis von Diamin (DMDAD) zu Bedeckungspolyisocyanat (N) beträgt 10,5 Äquivalente DMDAD zu 1,0 Äquivalente N. Nach einer halbstündigen Rührzeit bei Raumtemperatur wird das desaktivierte feste Polyisocyanat abgesaugt und getrocknet. Das Filtrat ist praktisch diaminfrei. Das Polymer-umhüllte, feste Polyisocyanat stellt ein rieselfähiges, farbloses Pulver mit einem Erweichungsbereich von 118 bis 122° C dar.

1.4.2 Herstellung einer PU-bildenden Reaktivmischung

9,0 g dieses Polyharnstoff-umhüllten Produktes 1.4.1 werden mit 50 g AAPE verrührt. Die Mischung ist bei Raumtemperatur über Monate lagerstabil. In einer Probe wird der Aufdickungspunkt dieses Systems zu 120 bis 130° C bestimmt.

1.4.3 Abgewandeltes Verfahren zur Polymerumhüllung (Diamin in Polyetheramin gelöst)

9,0 g des in Beispiel 1.1 beschriebenen Adduktes aus TT und N werden mit 50 g des aromatischen Aminopolyethers AAPE vermischt und dieser Mischung werden 0,15 g des Diamins DMDAD zugesetzt. Es bildet sich eine Polymerumhüllung auf den festen Polyisocyanatteilchen und die Mischung verhält sich bei Raumtemperatur über Monate lagerstabil und zeigt in einer Probe einen Aufdickungspunkt dieses Systems von 115 bis 120° C.

1.4.4 Verfahrensabwandlung (Zumischung der bedeckten Polyisocyanate zu einer Lösung von Diamin in Aminopolyether)

Die gemäß Beispiel 1.1, 1.2 und 1.3 erhaltenen, festen, feinteiligen bedeckten Polyisocyanate werden mit AAPE vermischt, in dem vorher die folgenden Diamine a), b) oder c) in der angegebenen Menge gelöst worden waren (siehe Rezepturen a) bis c)).

Nach ca. 1 Stunde Lagerzeit bei Raumtemperatur werden die jeweiligen Aufdickungspunkte der PU-Reaktivsysteme bestimmt.

| Rezepturen | a | b | c |
|---|---|---|---|
| Aminopolyether AAPE | 50 g | 50 g | 50 g |
| Diamin | 0,2 g (DMDAD) | 0,05 g (Ethylendiamin) | 0,10 g Diethylentriamin |
| Polyisocyanat-bedecktes Polyisocyanat (nach Beispiel) | 9 g (1.1) | 7 g (1.2) | 6 g (1.3) |
| Lagerstabilität bei RT | stabil | stabil | stabil |
| System-Aufdickungspunkt | 110°C | 80-90°C | 60-70°C |

**Beispiel 2**

2.1 Erfindungsgemäßes Verfahren

Einer Suspension von 25 g TT in 150 g Hexan werden 2,6 g N (21,3 % NCO) zugesetzt und die Mischung eine halbe Stunde bei Raumtemperatur gerührt. Danach erfolgt Zugabe von 1,5 g DMDAD zum mit N bedeckten, festen Polyisocyanat TT. Nach einer Stunde wird das so polymermodifizierte, feste Polyisocyanat (mit Polyharnstoffhülle) abgesaugt, mit Petrolether gewaschen und getrocknet. Im Filtrat ist kein aliphatisches Diamin nachweisbar (Titration mit 0,1 n HCl)

Um das Maß der Desaktivierung des TT zu bestimmen, werden 17 g des erhaltenen, festen, Polymerumhüllten Polyisocyanats mit 100 g AAPE vermischt und der Aufdickungspunkt dieses Systems zu 85 bis 90°C bestimmt.

Das in dünnen Schichten auf Textilien oder feste Unterlagen aufgetragene System vernetzt innerhalb weniger Minuten bei 120°C zu einer elastomeren Polyurethan-Beschichtung.

Die Mischung verhalt sich dagegen bei Raumtemperatur über mindestens mehrere Wochen lagerstabil.

2.2 Vergleichsversuch nach dem Stand der Technik durch direkte Umsetzung von Diamin mit dem feinteiligen festen Polyisocyanat.

Obiger Versuch wird ohne Zusatz von biuretisiertem Hexandiisocyanat (N) durchgeführt. Die Aufarbeitung und die Bestimmung des Aufdickungspunktes erfolgen wie in Verfahren 2.1 beschrieben.

Nach dem Absaugen des nach dem Stand der Technik durch Reaktion des Diamins mit den NCO-Gruppen an der Oberfläche der festen Polyisocyanatteilchen selbst desaktivierten Diisocyanats kann im Filtrat (mittels HCl-Titration) noch 1,05 g des aliphatischen Diamins (DMDAD) bestimmt werden. Das bedeutet, daß ca. 26 mMol aliphatisches Diamin (DMDAD) pro Mol festes Diisocyanat abreagiert sind und der überwiegende Anteil (ungefähr 70 %) des Diamins noch in freier Form im Lösungsmittel vorliegt.

Das abgesaugte, modifizierte Polyisocyanat wird mit Lösungsmittel gewaschen und ist nunmehr frei von noch überschüssigen, nicht umgesetzten aliphatischen Diaminen (DMDAD).

Im Testversuch mit aromatischen Aminopolyether (AAPE) wird nur ein Aufdickungspunkt von kleiner als 50°C gemessen. Die Mischung ist über Nacht bei Raumtemperatur bereits so aufgedickt, daß sie nicht mehr verarbeitbar ist. Der Vergleichsversuch zeigt, daß die erfindungsgemäß hergestellte Polymerumhüllung mittels eines per Bedeckung aufgebrachten Polyisocyanats B und dessen Abreaktion mit Diamin D wirkungsvoller ist als eine Desaktiverungsreaktion nach dem Stand der Technik an der Oberfläche der festen Polyisocyanatteilchen selbst. Die unzureichende Hülle nach dem Stand der Technik führt zu niedrigen Aufdickungspunkten und Instabilität der PU-Reaktivmischung.

**Beispiel 3**

3.1

Bedeckung

Zu einer Suspension von 100 Teilen feinteiligem (mittlere Korngröße ca. 10 µm) TDIH in 520 Teilen Hexan läßt man unter Rühren 3,2 Teile einer 50 %-igen Lösung von N (NCO-Gehalt 21,3 %) in Toluol langsam zulaufen.

3.2 Polymerumhüllungsreaktion

Nach ca. 5 Minuten intensivem Rühren gibt man zur Dispersion, gemäß 3.1. erhalten, 1,92 Teile einer 50 %-igen Lösung von DMDAD in Toluol zu und rührt ca. 30 Minuten nach. Das durch eine Polyharnstoffhülle (aus N und DMDAD) geschützte TDIH wird abgesaugt, mit Hexan gewaschen und getrocknet.

3.3 PU-Reaktivmischung

19 Teile des polymerbedeckten Harnstoffdiisocyanates, nach 3.2 erhalten, werden mit 100 Teilen des Aminopolyethers AAPE zu einer stabilen Dispersion verrührt, die über längere Zeit bei Raumtemperatur lagerstabil ist. Diese Reaktivmischung weist einen System-Aufdickungspunkt von 80 bis 90° C auf.

Das sorgfältig entgaste Reaktivsystem (ca. 130 g) wird in eine 20 x 20 cm große, flache, kalte Form gegossen und in einem 120° C heißen Ofen 4 Stunden lang ausgeheizt.

Man erhält eine ca. 3 mm dicke Elastomer-Probeplatte mit folgenden mechanischen Eigenschaften:

| | | |
|---|---|---|
| Oberflächenhärte: | Shore A: 93 | |
| | Shore D: 42 | DIN 53 505 |
| Zugfestigkeit | 13,8 MPa | DIN 53 504 |
| Reißdehnung | 340 % | DIN 53 504 |
| Weiterreißfestigkeit | 34,5 KN/m | DIN 53 515 |
| Elastizität | 53 % | DIN 53 512 |

**Beispiel 4**

In situ-Herstellung eines sehr feinteiligen, festen, stabilisierten Polyisocyanates.

4.1 in situ-Herstellung eines sehr feinteiligen Polyisocyanats (TDIH)

In 800 ml DIP werden 174 g (1 Mol) TDI und 12,6 g (0,7 Mol) Wasser vorgelegt und bei Raumtemperatur intensiv gerührt. Nach wenigen Minuten beginnt eine allmählich sich steigernde $CO_2$-Entwicklung und das Reaktionsgemisch erwärmt sich nach und nach auf ca. 35° C. Die Reaktionsmischung wird mit Hilfe eines Wasserbades auf 50° C erwärmt, so daß die Umsetzung nach insgesamt 2 Stunden beendet ist. (Ende der $CO_2$-Entwicklung; insgesamt ca. 12 l $CO_2$ (unkorrigiert)).

Wie ein Parallelversuch zeigt, enthält das Lösungsmittel noch 0,02 Mol nichtumgesetztes TDI. Das isolierte Festprodukt weist einen NCO-Gehalt von 25,8 Gew.-% auf.

4.2 Polymerbedeckung des TDIH

Zu der Dispersion des in sehr feinteiliger Form in situ angefallenen TDIH werden nach Reaktionsende unter Rühren zunächst 4,44 g (0,02 Mol) IPDI und nach ca. 20 Minuten 9,52 g (0,04 Mol) Diamin DMDAD, gelöst in 20 ml DIP, zugegeben (0,02 Mol Diamin für die Umsetzung mit IPDI und 0,02 Mol für die Bindung des noch in der Lösung nichtumgesetzten freien TDI). Es wird ca. 2 Stunden bei 40° C nachgerührt. Das polymerumhüllte feste TDIH wird abgesaugt und im Vakuumtrockenschrank bei 50° C getrocknet. Ausbeute: 163,4 g (93,3 % der Theorie), Erweichungsbereich 180 bis 190° C, NCO-Gehalt 22,5 Gew.-% (theoretisch 23,5 Gew.-% - unter Berücksichtigung der Polymerumhüllung).

4.3 Polymerumhüllte Polyisocyanat-Dispersion in Weichmachern und ihre Verwendung

400 ml der bei der Reaktion nach 4.2 erhaltenen Dispersion des polymerumhüllten Polyisocyanats in DIP (NCO-Gehalt 4,52 Gew.-%) werden mit 260 g Dioctylphthalat-Weichmacher vermischt. Anschließend wird der Diisopropylether am Rotationsverdampfer entfernt. Man erhält 326,4 g einer Dispersion des polymerumhüllten Polyisocyanats mit einem NCO-Gehalt von 5,04 Gew.-%.

44,8 Teile dieser Dispersion werden mit 50 Teilen AAPE vermischt. Die erhaltene Reaktivmischung zeigt bei Raumtemperatur keine Viskositätserhöhung bei mehrmonatiger Lagerung. Der Aufdickungspunkt dieses Systems liegt bei 85° C.

4.4 (Vergleichsversuch)

Zum Vergleich wurden 20,6 g nichtstabilisierter, gemahlener (ca. 10 µm mittlerer Teilchendurchmesser) TDIH, NCO-Gehalt 24,5 Gew.-%, in 79,4 Teilen Dioctylphthalat dispergiert. Wird diese Dispersion in obigem Stabilisierungstest eingesetzt, so wird eine instabile Reaktivmischung erhalten, die innerhalb von ca. 1 Stunde aufdickt.

**Beispiel 5**

Verwendung der polymerumhüllten Polyisocyanate zur Herstellung eines Elastomers

100 Teile AAPE werden mit 20,1 Teilen des in Beispiel 4.2 hergestellten, polymerumhüllten festen Diisocyanats, Kennzahl $NCO:NH_2 = 1{,}25$, intensiv mit einem Schnellrührer zu einer Suspension vermischt und anschließend im Hochvakuum ca. 15 Minuten entgast.

Von der erhaltenen PU-Reaktivsuspension wird ein schmaler Streifen auf eine Koflerbank aufgetragen und nach bestimmten Zeiten festgestellt, ab welcher minimalen Temperatur eine elastische Verfestigung eingetreten ist.

| Zeit (min) | elastische Verfestigung ab (°C) |
|---|---|
| 3 | ⩾ 85 |
| 5 | ⩾ 82 |
| 10 | ⩾ 80 |

Das Material wird in eine 20 cm x 20 cm x 0,5 cm große, flache Metallform gegossen und 4 Stunden bei 120°C ausgehärtet.

Man erhält eine Elastomerprobeplatte mit folgenden mechanischen Eigenschaften:

| | | |
|---|---|---|
| Zugfestigkeit | 12,1 MPa | DIN 53 504 |
| Reißdehnung | 200 % | DIN 53 504 |
| Weiterreißfestigkeit | 26,4 KN/m | DIN 53 515 |
| Shore Härte nach DIN 53 505 | 92 A und 43 D | |
| Elastizität | 52 % | DIN 53 512 |

**Beispiel 6**

Polymerumhüllung mit verschiedenen Polyisocyanaten B und verschiedenen Reaktionsmedien C

Diese Beispiele zeigen, in welchem Umfange die Stabilisierung eines festen Polyisocyanates A nach dem erfindungsgemäßen Verfahren von der chemischen Konstitution, der Löslichkeit und der Ausfällungsgeschwindigkeit der aus den flüssigen Polyisocyanaten B und den aliphatischen Diaminen D entstandenen Polyharnstoffe in verschiedenen Reaktionsmedien C abhängt. Aus nachstehender Übersicht sind diese unterschiedlichen Stabilisierungseffekte deutlich erkennbar. Die Versuche werden mit feinteiligem, festen Polyisocyanat A, dispergiert in 15 g "Lösungsmittel" C, x g (siehe Tabelle) flüssigem Polyisocyanat B und einer Mischung von 0,68 g Diamin DMDAD, 50 g aromatischem Aminopolyether AAPE und Katalysator durchgeführt.

**Tabelle**

Umhüllung von TT mit verschiedenen Polyisocyanaten B in verschiedenen Lösungsmitteln; Zustand und Aufdickungspunkt der PU-Reaktiv-mischung

| Polyisocyanat B) (in g) | Hexan | DIP | PPG-Etherpolyol | Toluol | Dioctylphthalat |
|---|---|---|---|---|---|
| IPDI (x = 0,65 g) | flüssig AP: 130-140° C | flüssig : 140° C | flüssig : ~150° C | aufgedickt (24 h) - | aufgedickt (24 h) - |
| HDI (x = 0,48 g) | schwach thixothrop AP: 140-150° C | flüssig ~150° C | flüssig ~160° C | aufgedickt - | aufgedickt - |
| N (21,3 % NCO) (x = 1,12 g) | flüssig AP: 170° C | flüssig ~170° C | flüssig ~150° C | aufgedickt - | aufgedickt - |
| HDI-Trim. (21,7 % NCO) x = 1,1 g | flüssig AP: ~160° C | flüssig ~160° C | flüssig ~160° C | aufgedickt - | aufgedickt - |
| HDI/Dipropylenglykol-(2 : 1)-Addukt x = 1,27 g | flüssig AP: ~110-120° C | - | - | - | - |
| TDI/Trimethylolpropan-(3 : 1)-Addukt x = 1,37 g | flüssig AP: ~100-110° C | - | - | - | - |
| Vergleich ohne | flüssig AP: ~160° C | flüssig ~145° C | flüssig ~150° C | flüssig ~150° C | flüssig ~160° C |

In der Tabelle werden in Abhängigkeit von den angewendeten Lösungsmitteln C der Einfluß der Menge und Art der Polyisocyanate B auf das Aufdickungsverhalten bzw. den Aufdickungspunkt der erhaltenen PU-Reaktivmischung wiedergegeben.

Als Vergleichsversuch wird eine Reaktion durchgeführt, wo die Polymerumhüllung durch das separat zugegebene Polyisocyanat B unterblieben ist (Verfahren nach dem Stand der Technik). In der Tabelle wird unter A.P. der System-Aufdickungspunkt wiedergegeben. Bei ungenügender Stabilisierung erfolgt eine alsbaldige Aufdickung der Reaktivmischung, die dann nicht weiterverarbeitbar ist.

Wie aus der Tabelle hervorgeht, sind höherpolare Lösungsmittel, welche eine zu gute Lösungswirkung auf das Polyisocyanat B ausüben und somit nicht zu einer Bedeckung des Polyisocyanats A (TT im vorliegenden Falle) führen, praktisch als Komponente C ungeeignet.

**Beispiel 7**

Zeitabhängigkeit der Polyisocyanat-Bedeckung

7.1 Polyisocyanat-Bedeckung - kurzzeitig

Zu einer Suspension von 17 g TT in 30 g Dioctylphthalat werden 1,3 g IPDI zugesetzt. Direkt anschließend (3 Minuten) werden 1,3 g DMDAD und 100 g AAPE eingerührt. Der Aufdickpunkt dieser PU-Reaktivmischung liegt unter 50° C; nach einigen Stunden Stehen der Reaktivmischung bei Raumtemperatur erfolgt eine starke Aufdickung.

7.2 Polyisocyanat-Bedeckung - nach Standzeit

Werden dagegen den obengenannten Suspensionen von TT in Dioctylphthalat und IPDI erst nach einem Zeitraum von 2 bis 3 Stunden 1,3 g Diamin DMDAD und 100 g aromatischer Aminopolyether AAPE zugesetzt, so erhält man nunmehr eine bei Raumtemperatur lagerstabile Mischung mit einem Aufdickungspunkt des Systems von 70 bis 75° C. Dieser Ansatz besitzt nach längerem Stehen ein leicht thixotropes Verhalten.

Dieses Ergebnis wird so gedeutet, daß das feste Polyisocyanat TT erst allmählich mit dem flüssigen IPDI in Wechselwirkung (unter Adsorption bzw. Absorption) tritt. Das zugegebene Diamin DMDAD erzeugt dann den für die retardierte Reaktivität der Mischung verantwortlichen Polyharnstoff auf der festen Polyisocyanat-Teilchenoberfläche.

**Kommentar:**

Der obige Zeiteffekt wird im allgemeinen ausgeprägt nur dann beobachtet, wenn ein (niedermolekulares) Diisocyanat B wie z. B. HDI oder IPDI bzw. TDI und ein relativ polares Lösungsmittel, wie Toluol oder Dioctylphthalat, bei der Polyisocyanat-Bedeckungsstufe eingesetzt wird.

**Beispiel 8**

Zur Erhärtung der Tatsache, daß der bei der externen Reaktion eines flüssigen Polyisocyanats B und eines Diamins D in Gegenwart eines festen, feinteiligen Polyisocyanats A entstandene Polyharnstoff auf der Oberfläche des festen Polyisocyanats A gebildet wird, und insbesondere daß die dadurch verursachte Desaktivierung von A ohne Mitreaktion der NCO-Gruppen des festen Polyisocyanats mit dem Diamin abläuft, wurden folgende Versuche durchgeführt. Dabei wurde als aliphatisches Diamin DMDAD und als Komponente B Polyisocyanat N verwendet.

8.1 Erfindungsgemäße Verfahren

Nachstehende Ausgangangskomponenten werden in der angegebenen Reihenfolge vermischt und der Aufdickungspunkt der Reaktivmischung als Maß für die Lagerstabilität bestimmt.

| | | |
|---|---|---|
| 15 | g | eines lineraren PPG-Ethers (OH-Zahl 56) |
| 9,0 | g | TT |
| 0,34 | g | N werden 30 Min. gerührt, dann mit 0,2 g Diamin DMDAD, |
| 50 | g | AAPE und |
| 0,05 | g | Pb-octoat/Lösung in Ligroin, ca. 57 %-ige Lösung, vermischt. |

Der Aufdickpunkt dieser Mischung beträgt 130 bis 140° C (bestimmt nach einer Lagerzeit von 1 Stunde bei RT nach Vermischung).

8.2 Vergleichsversuch zu Beispiel 8.1

Obiger Ansatz wurde ohne Zugabe von 0,32 g Polyisocyanat durchgeführt. Nunmehr liegt der Aufdickungspunkt der Mischung bei 85 bis 90° C, also deutlich tiefer als der nach dem erfindungsgemäßen Verfahren 8.1 gemessene Aufdickungspunkt.

Dieses Ergebnis deutet bereits darauf hin, daß die nach dem Verfahren 8.1 beobachtete Desaktivierung des festen Polyisocyanats gegenüber den aromatischen $NH_2$-Gruppen im Aminopolyether nicht dadurch erklärt werden kann, daß das aliphatische Diamin C sowohl mit dem in flüssiger Phase (Polyisocyanat B) als auch dem in heterogener Phase (Polyisocyanat A) vorliegenden NCO-Gruppen gleichmäßig reagiert. Wäre dies der Fall, so sollte der Aufdickungspunkt (die Lagerstabilität) der nach dem erfindungsgemäßen Verfahren 8.1 hergestellten Mischung deutlich unter dem der nach Verfahren B hergestellten Mischung liegen.

Infolge der Konkurrenzreaktion des aliphatischen Diamins mit dem flüssigen, NCO-gruppenhaltigen Polyisocyanat B müßte dann auch die Aminkonzentration geringer sein, als in Beispiel 8.2 beschrieben, und damit eine geringere Stabilisierung des festen Polyisocyanats als im Beispiel 8.2 herbeiführen.

Die deutlich erhöhte Stabilisierung nach dem erfindungsgemäßen Beispiel 8.1 wird dadurch erklärt, daß der auf der Teilchenoberfläche des festen Polyisocyanats A sich bildende Polyharnstoff aus dem Diamin D und dem flüssigen Polyisocyanat B einen wesentlich stärkeren Abschirmeffekt zeigt, als der aus dem Diamin D und dem festen Polyisocyanat A alleine entstandenen Polyharnstoff nach Vergleichsversuch 8.2.

**Beispiel 9**

Änderung des $NCO/NH_2$-Verhältnisses von Polyisocyanat B zu Verbindung D

Es werden nach dem erfindungsgemäßen Verfahren verschiedene Reaktionsmischungen gemäß Beispiel 8

hergestellt, wobei das Verhältnis Mol NCO aus dem Biuretpolyisocyanat B zu Mol $NH_2$ im Diamin D ein Äquivalent-Verhältnis B zu D von 0 bis 2,05 überdeckt. Die Menge des zugesetzten Diamins D (DMDAD) bleibt konstant, während die Menge des Biuretpolyisocyanates B in entsprechender Weise variiert wird.

| Zugesetze Menge an Biuret-poly-isocyanat B (g) | NCO (B)/$NH_2$ (D)-Verhältnis (Äquivalente) | System-Auf-dickungspunkt (° C) |
|---|---|---|
| 0 | 0 | 90 |
| 0,09 | 0,28 | 95 - 100 |
| 0,17 | 0,51 | 105 - 110 |
| 0,26 | 0,79 | 125 - 130 |
| 0,33 | 1,00 | 130 - 140 |
| 0,43 | 1,28 | 130 - 140 |
| 0,51 | 1,54 | 130 - 140 |
| 0,60 | 1,81 | 130 - 140 |
| 0,68 | 2,05 | 130 - 140 |

Zu beobachten ist eine deutliche Beeinflussung des Aufdickungspunktes bei zunehmender Menge an Biuret-Polyisocyanat B bis zum Erreichen des Äquivalenzpunktes (Molverhältnis NCO (B)/$NH_2$ (D) = 1 : 1), da in diesem Bereich die zur Desaktivierung des festen Polyisocyanats A verantwortliche Polyharnstoffmenge zunimmt. Die optimale Desaktivierung des festen Polyisocyanates A ist nach Erreichen des Äquivalentpunktes erreicht, so daß erhöhte Zusätze des NCO-Biuretpolyisocyanats 8 keinen Einfluß auf den Aufdickungspunkt des Systems mehr haben.

**Beispiel 10**

Stabilisierung eines Polyisocyanats durch Polyharnstoff-Umhüllung (Variation der Diamine)

10.1

In nachstehenden Reaktionsansätzen werden bei gleichbleibender Isocyanatkomponente B (IPDI) die Aminkomponenten D variiert. Das NCO/$NH_2$-Verhältnis (flüssiges Diisocyanat B zu aliphatischem Diamin D) bleibt dabei bei ca. 1. Es ergibt sich, daß die aus dem genannten flüssigen Diisocyanat B und den angegebenen Aminen D auf der Teilchenoberfläche des festen Polyisocyanates A gebildete Polyharnstoffhülle zu einer völlig ausreichenden Desaktivierung des festen Polyisocyanats gegenüber H-aciden Verbindungen, wie sie zur Polyurethanherstellung verwendet werden, führt. Die Mischungen verhalten sich auch bei mehrmonatiger Lagerung bei Raumtemperatur stabil.

Ansatz:

| | |
|---|---|
| 25 g | linearer Polypropylenglykolether (PPG-Ether) OH-Zahl 56 |
| 18 g | TT |
| 0,68 g | IPDI |
| x g | Diamin D (äquivalente Mengen zu IPDI). |

Nach 1 Stunde bei Raumtemperatur wird die Mischung weiter versetzt mit einer Lösung aus 25 g linearem PPG-Ether (siehe oben)

4,5 g 3,5-Diethyl-2,4/2,6(65/35)-diaminotoluol (DETA) und 0,5 g Pb-octoat-Lösung (wie vorstehend in Beispiel 8.1 beschrieben.

Als Diamine werden die in der folgenden Tabelle beschriebenen Di- oder Polyamine D eingesetzt:

| Diamin D | Menge x (g) | System-Aufdickungs-punkt (°C) |
|---|---|---|
| Ethylendiamin | 0,18 | 110° |
| Diethylentriamin | 0,29 | 120° |
| 2,5-Diamino-2,5-dimethylhexan | 0,4 | 110° |
| Isopharondiamin | 0,98 | 125° |
| DMDAD | 0,64 | 125° |

Die Verfestigung der Ansätze erfolgt oberhalb des Aufdickungspunktes und man erhält dann hochelastische Polyurethanformkörper mit folgenden mechanischen Wertebereichen:

| | |
|---|---|
| Härte (Shore A) | 90 bis 92 |
| Zugfestigkeit (MPa) | 8,9 bis 10 |
| Bruchdehnung (%) | 200 bis 240 |
| Weiterreißfestigkeit KN/m) | 14 bis 18 |
| Elastizität | 53 bis 55 |

**Beispiel 11**

In diesem Beispiel wird an Stelle von IPDI HDI-Trim (NCO-Gehalt 21,6 %), in Kombination mit verschiedenen Di- oder Polyaminen D in folgenden Reaktionsansätzen geprüft:

Ansatz:

| | |
|---|---|
| 15 g | linearer PPG-Ether, OH-Zahl 56 |
| 9,0 g | TT |
| 1,3 g | HDI-Trim |
| x g | Amine D |
| 50 g | aromatischer Aminopolyether AAPE |
| 0,5 g | Bleioctoat-Lösung in Waschbenzin |

| Amine | Menge (g) | System-Aufdickungs-punkt (°C) |
|---|---|---|
| Ethylendiamin | 0,18 | 80 |
| Diethylentriamin | 0,28 | 95 |
| 2,5-Diamino-2,5-dimethylhexan | 0,40 | 100 |
| IPDA | 0,48 | 90 |
| DMDAD | 0,69 | 95 |

Werden die bei Raumtemperatur gut verarbeitbaren, flüssigen, Einkomponenten-PU-Reaktivsysteme nach kurzem Entgasen im Vakuum in einer mit Trennmittel versehenen Form auf eine Temperatur von 110 bis 120°C erwärmt, so erhält man nach 1 bis 2 Stunden ein hochelastisches PU-Elastoomer mit folgenden mechanischen Eigenschaften. Das mechanische Werteniveau wird hierbei praktisch nicht von der Art der Polymerhülle beeinflußt.

| | |
|---|---|
| Härte (Shore A): | 92 |
| Zugfestigkeit (MPa): | 18,5 |
| Bruchdehung (%): | 420 |
| Weiterreißfestigkeit (KN/m): | 39,2 |
| Elastitizät (%) | 60 |

**Beispiel 12**

Zu einer Suspension von 9 g TT in 15 g eines linearen Polypropylenglykolethers (Molekulargewicht 2000) werden 1,12 g N zugesetzt. Nach kurzer Zeit erfolgt dann unter Rühren die Zugabe der unten beschriebenen Verbindungen in der angegebenen Menge. Anschließend werden 50 g AAPE zugesetzt. Nach einer Standzeit von 1 Stunde wird dann der Aufdickungspunkt des Systems bestimmt.

| Verbindung D | Menge (g) | Aufdickpunkt (°C) |
|---|---|---|
| 1,2-Dimethyl-tetrahydropyrimidin | 0,3 | 100° |
| 1,5-Diazabicyclo (4,3,0)-non-5-en | 0,32 | 110° |
| Tetramethylguanidin | 0,3 | 100° |
| Hydrazinhydrat | 0,2 | 70° |
| ß-Semicarbazidopropionsäurehydrazid | 0,45 | 70° |

($H_2N$-NH-CO-NH-$CH_2$-$CH_2$-CO-NH-$NH_2$)

**Beispiel 13**

Beeinflussung der Lagerstabilität (Aufdickungspunkt) eines Einkomponenten-PU-Reaktivsystems durch Zusatz von freien Isocyanaten

13.1 Erfindungsgemäßes Verfahren

Nachstehende Ausgangskomponenten werden in der angegebenen Reihenfolge vermischt und der Aufdickungspunkt als Maß für die Lagerstabilität der PU-Reaktivmischung nach 1 Stunde Lagerzeit bestimmt.

| | |
|---|---|
| 30 g | linearer PPG-Ether, OH-Zahl 56 |
| 22 g | TT |
| 0,55 g | N (NCO = 21,3 %) |
| 0,2 g | DMDAD |
| 100 g | aromatischer Aminopolyether (AAPE) |
| 0,1 g | Pb-octoat-Lösung (siehe Beispiel 8.1). |

Der Aufdickungspunkt der PU-Reaktionsmischung beträgt 100° C.

13.2 Vergleichsversuch (keine Bedeckung mit Polyisocyanaten B, sondern Stabilisierung nur durch Reaktion der festen Polyisocyanate A mit aliphatischen Diaminen)

Hierbei wurde durch geringfügige Änderung der Menge an zugesetztem aliphatischem Diamin ein zu 13.1 vergleichbarer Aufdickungspunkt eingestellt:

| | |
|---|---|
| 30 g | PPG-Ether, OH-Zahl 56, |
| 22 g | TT |
| 0,3 g | DMDAD |
| 100 g | AAPE |
| 0,1 g | Pb-octoat-Lösung (siehe Beispiel 8.1). |

Der Aufdickungspunkt dieses Systems beträgt 103° C.

Werden zu den nach Verfahren 13.1 und 13.2 hergestellten Einkomponenten-Reaktivsystemen zusätzlich freie Isocyanate (z. B. TDI) zugesetzt, so beobachtet man bei unten stehenden Mengen folgende Änderungen der Aufdickungspunkte:

| Verfahren | TDI-Zugabe g/150 g Einkomponentenmischung | | | | |
|---|---|---|---|---|---|
| | 0 | 0,2 | 0,3 | 0,4 | 0,6 |
| 13.1 erfindungsgemäß | 100 | 95 | 90 | 80 | 85 |
| 13.2 Stand der Technik | 103 | 60 | 50 | nicht mehr lagerstabil | |

**Beispiel 14**

Modifizierung des Einkomponentensystems durch nachträglichen Zusatz von freiem Isocyanat (Vorverlängerungsreaktionen in Einkomponenten-Systemen)

14.1 Erfindungsgemäßes Verfahren

Zu einer Suspension von 15 g TT in 30 g eines linearen Polypropylenglykolethers (OH-Zahl 56) werden 0,64 g N und kurze Zeit danach 0,4 g Diamin (DMDAD) zugesetzt. Anschließend erfolgt die Zugabe von 100 g eines aromatischen Aminopolyethers mit einer NH-Zahl 41,5 (Molmasse ca. 2400) und 0,1 g einer 57 %-igen Lösung von Pb-II-2-Ethylhexanoat in Waschbenzin. Man erhält eine Viskosität von 10 000 bis 12 000 mPa.s/Raumtemperatur. Um nunmehr die gewünschte Vorverlängerung des Aminopolyethers zu einem höhermolekularen Aminopolyether vorzunehmen, werden 3,6 g 2,4-TDI in die Reaktionsmischung eingerührt. Es erfolgt allmähliche Aufdickung (Reaktion des freien TDI mit Aminopolyether) und man erhält nach einigen Stunden ein plastisches, hochviskoses System von pastenartiger Konsistenz. Diese Masse verhält sich bei Raumtemperatur lagerstabil und kann zu einem beliebigen Zeitpunkt bei Temperaturen von 110 bis 130°C zu einem hochelastischen Polyurethanmaterial hitzevernetzt werden.

14.2 Vergleichsversuch

Die Zugabe der Einzelkomponenten erfolgt in der Reihenfolge wie in 14.1 beschrieben; der Unterschied ist jedoch, daß kein Biuret-polyisocyanat (N) mitverwendet wurde. Nach Zugabe von 3,6 g TDI erhält man auch hier ein hochviskoses Voraddukt, das sich aber bei Raumtemperatur nicht lagerstabil verhält. Bereits nach ca. 1 Tag ist die Masse so aufgedickt, daß eine weitere Verarbeitung unter üblichen Bedingungen unmöglich ist.

**Beispiel 15**

Verhalten des Aufdickungspunktes in Polyurethan-Reaktivsystemen bei voluminösen Formkörpern

15.1 Erfindungsgemäß

90 g TT werden in 100 g eines linearen Polypropylenglykolethers (Molekulargewicht 2000) dispergiert und 1,2 g N untergemischt. Nach 30 Minuten wird eine Mischung aus 0,8 g Diamin DMDAD, 500 g AAPE und 0,5 g Bleioctoat-Lösung (siehe Beispiel 8.1) untergemischt. Die PU-Reaktivmischung ist lagerstabil bei Raumtemperatur. Sie besitzt einen Aufdickungspunkt von 100°C.

Der Reaktionsansatz wird vollständig in eine 1 Liter fassende Blechdose (Durchmesser 12 cm, Höhe 10 cm) gegossen und in einem Heizschrank mit einer Innentemperatur von 120°C gelagert. Die Verfestigung des Reaktionsgemisches schreitet mit zunehmender Temperatur von außen nach innen fort.

Während der allmählichen Temperaturerhöhung der Reaktionsmischung werden in der angegebenen Zeit jeweils Proben aus den inneren, noch flüssigen Bestandteilen des aushärtenden Reaktionsgemisches entnommen und an diesen Proben wird deren jeweiliger Aufdickungspunkt separat bestimmt. (siehe Tabelle)

| Zeit/Stunden | Temperatur flüssigen Kerns | des Aufdickungspunkt (°C) |
|---|---|---|
| 0 | Raumtemperatur | 100 |
| 1/2 | 40 | 105 |
| 1 | 60 | 100 |
| 2 | 75 | 105 |
| 4 | 85 | 105 |

Wie die Tabelle zeigt, ändert sich der Aufdickungspunkt der erfindungsgemäßen Mischung während der Verfestigungsphase praktisch nicht. Nach 6 bis 8 Stunden ist der Reaktionsansatz vollständig verfestigt und der Probekörper zeigt nach dem Entformen eine glatte und unbeschädigte Außenhülle und über seinen Querschnitt eine gleichmäßige Struktur.

15.2 Vergleichsversuch

Der obige Versuch wird ohne Zugabe von N durchgeführt. Hierbei entsteht durch Reaktion des Diamins DMDAD mit den Isocyanatgruppen an der Oberfläche des festen Polyisocyanats (TT) selbst eine Polymerhülle.

Der Aufdickungspunkt dieses Reaktivsystems beträgt 90°C. Unter den bereits genannten Bedingungen der Bildung des Formkärpers und der Ausheizung werden an Proben des flüssigen Kerns folgende Ergebnisse für den Aufdickungspunkt erhalten:

| Zeit/Stunden | Temperatur flüssigen Kerns | des Aufdickungspunkt (°C) |
|---|---|---|
| 0 | Raumtemperatur | 90 |
| 1/2 | 40 | 95 |
| 1 | 60 | 110 |
| 2 | 75 | 125 |

Der Aufdickungspunkt der Reaktionsmischung ändert sich deutlich während der Verfestigung von den Randzonen her. Während der Ausheizphase reagiert das in der Mischung noch frei vorliegende aliphatischen Diamin mit dem festen Polyisocyanat, so daß mit langsam zunehmender Temperatur ein kontinuierlicher Anstieg des Aufdickungspunktes zu beobachten ist. Der innere Kern der Probe bleibt auch nach äußerer Verfestigung des Ansatzes noch lange flüssig. Bei weiterer Temperaturerhöhung erfolgt infolge thermischer Ausdehnung des noch nicht verfestigten flüssigen Materials an irgendwelchen Schwachstellen eine Aufsprengung der äußeren, bereits verfestigten Kruste, so daß hier flüssiges Material ausfließt Allmählich tritt dann bei weiterer Temperaturerhöhung Verfestigung ein und man erhalt ein Formteil, das neben Spannungsrissen auch noch eine völlig vernarbte Oberfläche aufweist.

**Beispiel 16**

16.1 Herstellung des polymerumhüllten Polyisocyanats

Zu einer Suspension von 200 g feinteiligem (mittlere Korngröße etwa 10 µm) TDIH in 1400 ml n-Hexan läßt man unter Rühren bei Raumtemperatur eine Lösung von 46,2 g eines NCO-Prepolymeren aus 3 Mol TDI und 1 Mol Trimethylolpropan (NCO-Gehalt 17,5 %) in 92 g Ethylglykoletheracetat langsam zulaufen. Nach 5 Minuten intensivem Rühren hat sich das zugesetzte NCO-Prepolymer auf dem festen TDIH niedergeschlagen. Zu dem so bedeckten TDIH läßt man nun unter Rühren eine Lösung von 19,04 g Diamin DMDAD in 100 ml n-Hexan zulaufen und rührt 4 Stunden bei 40°C nach. Das nun durch eine Polyharnstoff-Umhüllung geschützte TDIH wird abgesaugt, gewaschen und getrocknet.

16.2 PU-Reaktivsystem unter Verwendung eines aliphatischen Polyetherdiamins

16.2.1 Vorverlängerungsreaktion des Diamins (nicht erfindungsgemäß)

Zu 100 Teilen eines Polyoxypropylenetherdiamins mit aliphatischen $NH_2$-Gruppen (Molekulargewicht 2000) (Jeffamine® D-2000, der Firma Texaco) läßt man unter intensivem Rühren 4,44 Teile IPDI zulaufen. Nach 1-stündigem Entgasen bei 90°C erhält man ein Polyoxypropylenetherdiamin von nunmehr erhöhtem, mitterem Molekulargewicht 3777 und mit der Viskosität von 26 000 mPa.s bei 22°C.

16.2.2 PU-Reaktivmischung (erfindungsgemäß)

Man setzt diesem vorverlängerten Polyoxypropylenetherdiamin 15 Teile 2,4-/2,6-(65/35)-Diamino-3,5-diethyltoluol (DETA) zu und suspendiert in dieser Mischung 55 Teile des nach den obengenannten Methode (Beispiel 16.1) polymerumhüllten Diisocyanats. Nach dem Entgasen bei 50°C erhält man eine bis 50°C gut lagerstabile Suspension mit einer Viskosität von 300 000 mPa.s bei 22°C. Die Aufdickungstemperatur des PU-Reaktivsystems beträgt 92°C. Eine aus der PU-Reaktivsuspension durch Ausheizen hergestellte Elastomerprobe zeigt eine Härte von 62 Shore D.

16.3 Verwendung der PU-Reaktivmischung als Reaktivklebstoff

Die bei Raumtemperatur stabile, bei höherer Temperatur schnell abbindende, hitzehärtende Masse kann als Polyharnstoff-Reaktiv-Klebstoffmasse, welche eine gute Lagerfähigkeit bei Raumtemperatur und eine spontane Viskositätserhöhung bei relativ niedrigen Ausheiztemperaturen unter schneller Durchhärtung und verbesserten Klebeeigenschaften auch bei höheren Temperaturen, sowie verbesserten Adhäsionseigenschaften zeigt, verwendet werden.

16.3.1 Prüfvorschrift zur Ermittlung der Scherfestigkeit von Klebungen

Als Prüfmaterial dienen Streifen eines mit 30 % Glasfaser verstärkten Polyesterharzes (SMC) der Abmessung 20 mm x 40 mm x 4 mm und Eisenbleche der Abmessung 20 mm x 40 mm x 2 mm, die vor dem Klebstoffauftrag mit einem Schleifpapier der Körnung 280 leicht angeschliffen werden.

Die Streifen werden auf 120°C vorgewärmt. Nach Auftrag des Klebstoffs in einer Stärke von 0,2 mm werden zwei Streifen so zusammengelegt, daß eine überlappte Fläche von 10 x 20 mm entsteht. Diese Probekörper werden während der unten angegebenen Zeit bei 120°C gelagert. Unmittelbar darauf erfolgt die manuelle Prüfung der Scherfestigkeit, die bei 1 N/mm$^2$ ohne Trennung der Klebung nun eine Handhabung zur Weiterverarbeiung erlaubt. Nach 1-stündiger Lagerung der Prüfkörper bei Raumtemperatur wird die Scherfestigkeit nach DIN 53 283 mit einem Spindelvorschub von 100 mm pro Minute ermittelt.

16.3.2 Meßergebnisse

Die Ergebnisse sind in der Tabelle für die Klebung auf glasfaserverstärkten Polyesterharzen bzw. auf Eisen dargestellt. Die Scherfestigkeiten wurden nach 15 Minuten bei 120°C zunächst sofort, d.h. noch im heißen Zustand per Handprobe geprüft (siehe erste Spalten) bzw. nach Lagerung der Klebung nach 1 Stunde bei Raumtemperatur geprüft (siehe weitere Spalten).

**Tabelle**

| Klebstoffmischung nach Beispiel | Scherfestigkeit [N/mm$^2$] nach 15 Min./120°C sofort (heiß geprüft in Handprobe) | | Scherfestikeit [N/mm$^2$] nach 15 Min./120°C und nach Lagerung (1 Stunde bei Raumtemp.) | |
|---|---|---|---|---|
| | SMC | Eisen | SMC | Eisen |
| 16 | >1 | >1 | 9,5 | 15,0 |

**Beispiel 17**

Verwendung polymerumhüllter Polyisocyanate zur Herstellung von Einkomponentenpolyurethanelastomeren

In 2000 g eines linearen Polypropylenglykolethers des Molekulargewichts 2000 werden 111 g IPDI gelöst. Nach 40-stündigem Rühren bei 130°C und Entgasen erhält man ein vorverlängertes Polyetherdiol des Molekulargewichts 4222 und mit einer Viskosität von 3100 mPa.s bei Raumtemperatur.
In 100 g dieses Diols werden 14,8 g TT suspendiert und anschließend 1,59 g N zugefügt. Nach 5 Minuten intensivem Rühren hat sich das zugesetzte Biuretpolyisocyanat auf dem festen Diisocyanat als Bedeckung niedergeschlagen. Man rührt nun 0,43 g Diethylenglykol ein und rührt 30 Minuten bei Raumtemperatur nach. Nach Zusatz von 2,5 g 2,4-/2,6-(65/35)-Diamino-3,5-diethyltoluol-Isomerengemisch (DETA) und 0,2 g Bleioctoat-Lösung (siehe Beispiel 9.1) wird 1 Stunde bei 40°C entgast. Man erhält eine bis 40°C gut lagerstabile Suspension mit einer Viskosität von 7000 mPa.s bei 20°C und einem Aufdickungspunkt von 64°C. Nach Verfestigung und 2-stündiger Temperung bei 120° C erhält man ein opak erscheinendes Elastomer mit folgenden Eigenschaften:

| | | |
|---|---|---|
| Zugfestigkeit (MPa) | 4,5 | DIN 53 504 |
| Reißdehnung (%) | 200 | DIN 53 504 |
| Weiterreißfestigkeit (KN/m) | 8 | DIN 53 515 |
| Shore-Härte (A) | 62 | DIN 53 505 |
| Elastizität (%) | 34 | DIN 53 512 |

**Beispiel 18**

Verwendung zum Aufbau von Polyurethanharnstoffelastomeren auf Basis aromatischer und aliphatischer Aminopolyether

In 100 Teile eines aromatischen Aminopolyethers auf Basis eines Polyoxypropylenetherdiols (Molekulargewicht 2000) und Toluylen-2,4-diisocyanat (Molverhaltnis 1 : 2), hergestellt nach dem Verfahren gemäß DE-A-2 948 419, mit einer Aminzahl = 48,4 und einem Molekulargewicht von 2334, werden 2,0 Teile N (21,5 % NCO), emulgiert. In dieser Emulsion werden 26,3 Teile TT suspendiert, wobei sich die Tröpfchen des Biuretpolyisocyanats (N) auf dem festen, feinteiligen TT als Oberflächenbedeckung niederschlagen. Anschließend werden 1,2 Teile DMDAD zugegeben und zur Bildung der Polyharnstoffpolymerbedeckung 2 Stunden bei 40°C gerührt. Zuletzt werden 100 Teile eines aliphatischen Aminopolyethers zugegeben, der durch Umsetzung von 100 Teilen eines Polyoxypropylenetherdiamins vom Molekulargewicht 2000 (Jeffamine® 2000

der Firma Texaco, aliphatische Aminoendgruppen) mit 5,55 Teilen Isophorondiisocyanat hergestellt wurde.

Man erhält eine bis 50° C gut lagerstabile Einkomponentengießmischung mit einer Aufdickungstemperatur von 86° C und einer Viskosität von 20 000 mPa.s bei 22° C. Nach Verfestigung und 8-stündiger Temperung bei 120° C erhält man ein hochwertiges Elastomer mit den folgenden mechanischen Eigenschaften:

| | | |
|---|---|---|
| Zugfestigkeit (MPa) | 12 | DIN 53 504 |
| Reißdehung (%) | 600 | DIN 53 504 |
| Weiterreißfestigkeit (KN/m) | 29 | DIN 53 515 |
| Shore-Härte A | 77 | DIN 53 505 |
| Elastizität (%) | 42 | DIN 53 512 |

**Beispiel 19**

Abhängigkeit der Aufdickungstemperatur von der Menge an Polymerumhüllung

19.1 Erfindungsgemäßes Verfahren

Im folgendem Reaktionsansatz wurde die auf dem TT gebildete Menge an Polyharnstoff durch Variierung der Menge der Komponenten B (N) und D (DMDAD) in der angegebenen Weise variiert. Dabei wurde das $NCO/NH_2$-Verhältnis der beiden, die Polymerumhüllung ergebenden Komponenten bei ca. 1,1 gehalten, d.h. das Isocyanat liegt stets in einem geringen Überschuß vor.

Ansatz:

| | |
|---|---|
| 15 g | eines linearen Polypropylenglykolethers, Molekulargewicht 2000, |
| 9 g | TT |
| x g | N |
| y g | DMDAD |
| 50 g | AAPE |
| 0,05 g | Blei-octoat-Lösung in Waschbenzin (siehe Beispiel 8.1). |

In dem PU-Reaktivsystem ergeben sich folgende Aufdickungspunkte (gemessen nach 1 Stunde):

Reaktionskomponenten für Polymerumhüllung

| x (g) N | y (g) DMDAD | Aufdickungspunkt (° C) |
|---|---|---|
| 0,1 | 0,05 | 70 |
| 0,18 | 0,10 | 95 |
| 0,27 | 0,15 | 135 |
| 0,35 | 0,20 | 140 |
| 0,52 | 0,30 | 140 |
| 0,85 | 0,50 | 142 |

Bereits bei geringen Zusätzen beider Komponenten N/DMDAD erreicht man durch Polymerumhüllung eine genügende Lagerstabilität der Reaktivmischungen bei Raumtemperautr (Systemaufdickungspunkt ca. 70 bzw. 95° C). Ab einer Grenzkonzentration beider Verbindungen wird der Aufdickungspunkt der Reaktionsmischungen praktisch nicht mehr verändert. Die gebildete Hülle erscheint praktisch ausreichend zur Stabilisierung.

19.2 Vergleichsversuch (ohne Zusatz von N)

Zum Vergleich wird die Abhängigkeit des Aufdickungspunktes eines gleichartigen Reaktivsystems nach dem Stand der Technik getestet, indem man keine Bedeckung des feinteiligen festen Diisocyanats durchführt, sondern entsprechend dem Stand der Technik direkt auf der Oberfläche des TT eine Polyharnstoffbildung mit dem zugesetzten Diamin (DMDAD) durchgeführt. Der Ansatz ist derselbe wie in Beispiel 19.1, jedoch entfällt der Zusatz der x g an N, also der Komponente B. Die folgende Tabelle zeigt die Ergebnisse.

| y g / DMDAD | Aufdickungspunkt (°C) |
|---|---|
| 0 | nach ca. 15 Minuten Aufdickung |
| 0,05 | 50°, nach ca. 1 Stunde Aufdickung |
| 0,1 | 65 |
| 0,15 | 75 |
| 0,20 | 90 |
| 0,30 | 100 |
| 0,50 | 130 |

Es ist klar erkennbar, daß die desaktivierende Wirkung mit zunehmender Menge des aliphatischen Diamins zunimmt. Die Lagerstabilität ist also direkt proportional zur Diamin-Zugabe.

**Beispiel 20**

Zu einer Suspension von 100 g TT in 100 g Hexan wird bei Raumtemperatur eine Lösung von 7,8 g N in 5 g Toluol zugetropft. Es wird eine halbe Stunde nachgerührt, danach werden 4,5 g DMDAD zugesetzt. Nach 1 Stunde wird abgesaugt. Man erhält 108,5 g des mit Polyharnstoff umhüllten feinteiligen TT.

Von diesem Produkt werden 36 g in eine Schmelze eines linearen Polyesterdiols (Molekulargewicht 2000) aus Adipinsäure und Ethylenglykol bei 50 bis 60°C eingerührt. Weiterhin werden 0,4 g Formrez® UL-29 (S-haltiger Sn-Katalysator der Fa. Witco (USA)) zugesetzt. Danach läßt man die Schmelze erkalten.

Die Schmelze kann durch einen geeigneten Mahlprozeß in Granulatform überführt werden. Zu einem beliebigen Zeitpunkt kann die bei Raumtemperatur lagerstabile Reaktivmischung wieder aufgeschmolzen, in eine geeignete Form gebracht und bei 120°C durch Hitzestoß verfestigt werden. Man erhält nach der Entformung ein hochelastisches Polyurethan mit folgenden mechanischen Eigenschaften:

| | |
|---|---|
| Härte (Shore-A) | 82 |
| Zugfestigkeit (MPa) | 36,5 |
| Reißdehung (%) | 780 |
| Weiterreißfestigkeit (KN/m) | 52 |
| Elastizität (%) | 58 |

**Beispiel 21**

21.1 Polyisocyanat-bedecktes MDI-Dimer

Einer Suspension von 20 g eines niedermolekularen, dimeren 4-4'-Diisocyanatodiphenylmethans (Herstellung gemäß EP-A-71 898 durch vollständige Dimerisierung von MDI) in 50 g Diisopropylether werden unter Rühren 2 g N zugesetzt. Nach 1 Stunde wird das Festprodukt abgesaugt.

Man erhält 21,75 % eines pulverförmigen bedeckten Polyisocyanats mit einem NCO-Gehalt von 17,85 %.

21.2 Polymer-umhülltes MDI-Dimer

Einer Lösung von 0,15 g DMDAD in 100 g AAPE werden 20 g des mit Polyisocyanat bedeckten MDI-Dimers (21.1) zugesetzt und homogen vermischt. Nach 1 Stunde mißt man einen Aufdickungspunkt dieser Suspension von 95°C. Die Reaktivmischung verhält sich bei Raumtemperatur lagerstabil, verfestigt sich aber innerhalb kurzer Zeit bei 120 bis 130°C zu einem hochelastischen Polyurethan-Elastomeren.

**Beispiel 22**

In eine Suspension von 17,4 g TT in 100 g eines Polyoxypropylenetherdiols vom Molekulargewicht 2000 werden 1,0 g N eingerührt. Nach ca. 30 Minuten erfolgt ein Zusatz von 0,16 g Ethylenglykol. Dieser Mischung werden nach weiteren 30 Minuten 5,0 g 2,4-Diamino-3,5-di-ethyltoluol und 0,1 g Bleioctoat zugesetzt. Der Reaktionsansatz bleibt bei Raumtemperatur viskositätskonstant. Eine deutliche Viskositätserhöhung

(Aufdickung durch Polyharnstoffbildung aus dem dimeren TDI und dem aromatischen Diamin) erfolgt erst bei höherer Temperatur, z. B. 75°C. Der auf einer Kofler-Bank bestimmte Aufdickungspunkt beträgt 70°C.

Wird anstelle von 0,16 g Ethylenglykol 0,28 g Diethylenglykol verwendet, so beobachtet man unter den genannten Bedingungen einen Aufdickungspunkt von 60-65°C, d.h. auch diese Mischung verhält sich bei Raumtemperatur lagerstabil.

**Beispiel 23**

23.1 Herstellung eines Urethan-modifizierten Polyols:

In einer Mischung aus 670 g eines Polyoxypropylenetherdiols vom Molekulargewicht 1000 (1,34 OH-Äquivalente), 300 g eines Polyoxypropylenethertriols vom Molekulargewicht 450 (2,00 OH-Äquivalente), 30 g Ethylenglykol (0,968 OH-Äquivalente) und 0,5 g Blei-(II)-bis-ethylhexanoat (24 % Pb) werden bei Raumtemperatur 154 g TT (0,885 NCO-Äquivalente) suspendiert und die Suspension unter Rühren auf 120°C erwärmt. Das TT geht dabei in Lösung und reagiert innerhalb weniger Minuten mit dem Polyolgemisch ab (IR-Nachweis, Viskositätsanstieg). Nach 2-stündigem Entgasen bei 90°C erhält man eine leicht opake, vorverlängerte Polyolmischung mit einer Viskosität von 35 000 mPas bei 22°C bzw. 500 mPas bei 70°C.

Anstelle von dimerem Toluylen-diisocyanat können auch 110,6 g (0,885 NCO-Äquivalente) MDI verwendet werden.

23.2 Polymerbedeckung von TT mit einem Polymeren aus N und DMDAD.

Man suspendiert nun bei Raumtemperatur in dem Urethanmodifizierten Polyol 68,4 g (3,93 Äquivalente NCO) TT und emulgiert nach 15 Minuten 17,3 g N (0,087 NCO-Äquivalente) ein. Das emulgierte N schlägt sich auf den suspendierten, festen Teilchen des TT nieder. Nach 10 Minuten Rühren bei Raumtemperatur setzt man unter weiterem Rühren 10,0 g DMDAD (0,084 $NH_2$-Äquivalente) zu. Nach weiteren 30 Min. Rühren bei Raumtemperatur entgast man 1 Stunde bei 50°C und erhält so eine bei Raumtemperatur gut lagerfähige Einkomponenten-PU-Klebstoffmasse mit einer Viskosität von 100 000 mPas bei 23°C.

Bei 100 bis 130°C härtet sie rasch zu einem zäh-elastischen, harten Polyurethan aus.

**Patentansprüche**

1. Verfahren zur Herstellung von festen, feinteiligen, durch eine Oberflächenhülle in ihrer Reaktivität retardierten Polyisocyanaten, dadurch gekennzeichnet, daß man

a) in einer ersten Stufe

- ein festes, feinteiliges Polyisocyanat A mit einem Schmelzpunkt oberhalb 38°C und einer Teilchengröße zwischen 0,5 und 200 µm mit
- einem in flüssiger Form vorliegenden, von A verschiedenen Polyisocyanat B, in einer Menge von 0,05 bis 50 Gew.-Teilen B pro 100 Teile A, in
- Suspension in einem Suspensionsmittel C, wobei C so ausgewählt ist, daß A nicht gelöst wird und B so schwer löslich ist, daß seine adsorptive Abscheidung auf A nicht verhindert wird, bei Temperaturen unterhalb des Schmelzpunktes von A oberflächlich bedeckt und danach gegebenenfalls C entfernt,

b) in einer zweiten Stufe

- das die Oberfläche der Polyisocyanatteilchen A bedeckende Polyisocyanat B mit mindestens einer mehrwertigen, in flüssiger Form vorliegenden, gegenüber Isocyanatgruppen reaktiven Verbindung D in Mengen von mindestens 0,5 Äquivalenten D pro Äquivalent B,
- gegebenenfalls in Suspension in einem Suspensionsmittel C, unter Ausbildung einer Hülle aus B und D um A umsetzt und

c) gegebenenfalls das Suspensionsmittel C entfern.

2. Verfahren nach Anspruch 1, dadurch gekennzeichnet, daß das Suspensionsmedium der ersten Stufe entfernt wird und die Umsetzung mit der Komponente D in einem anderen Suspensionsmedium erfolgt.

3. Verfahren nach Ansprüchen 1 oder 2, dadurch gekennzeichnet, daß man als Suspensionsmedium C aliphatische Kohlenwasserstoffe und/oder längerkettige Dialkylether und/oder Weichmacher und/oder höhermolekulare Polyole einsetzt.

4. Verfahren nach Ansprüchen 1 - 3, dadurch gekennzeichnet, daß man als Komponente A aromatische Polyisocyanate, vorzugsweise dimere, trimere Diisocyanate und/oder Harnstoffdiisocyanate einsetzt.

5. Verfahren nach Ansprüchen 1 - 4, dadurch gekennzeichnet, daß man als Komponente B aliphatische, cycloaliphatische Polyisocyanate, und/oder NCO-Prepolymere auf Basis höhermolekularer Polyhydroxylverbindungen verwendet.

6. Verfahren nach Ansprüchen 1 - 5, dadurch gekennzeichnet, daß man als Komponente D aliphatische oder cycloaliphatische Di- oder Polyamine, Hydrazin, Alkylhydrazine, N,N-Dialkylhydrazine und/oder Di- oder Polyhydrazidverbindugnen, und/oder offenkettige, mono- oder bicyclische Amidine oder Guanidine mit keinem, einem oder zwei gegenüber NCO reaktiven H-Atomen einsetzt.

7. Verfahren nach Ansprüchen 1 - 6, dadurch gekennzeichnet, daß die Verbindungen B und D unter Ausbildung einer Polymerhülle reagieren.

8. Umhüllte, feste, feinteilige Polyisocyanate retardierter Reaktivität, erhältlich nach dem Verfahren nach Ansprüchen 1 - 7, gegebenenfalls suspendiert in höhermolekularen Polyolen und/oder höhermolekularen Polyaminen.

9. Verwendung der Polyisocyanate nach Anspruch 8 als Polyisocyanatkomponente zur Herstellung von Polyurethanen.

10. Lagerstabiles Einkomponenten-Reaktivsystem, enthaltend Polyisocyanate nach Anspruch 8, höhermolekulare Polyhydroxyl- und/oder Polyaminoverbindungen, gegebenenfalls niedermolekulare Kettenverlängerungsmittel, gegebenenfalls Polyurethankatalysatoren und gegebenenfalls übliche Hilfs- und Zusatzstoffe.

**Claims**

1. Process for the preparation of solid, finely divided polyisocyanates which are retarded in their reactivity by a surface shell, characterised in that

a) in a first stage,
- a solid, finely divided polyisocyanate A having a melting point above 38°C and a particle size of from 0.5 to 200 µm is covered on its surface at temperatures below its melting point with
- from 0.05 to 50 parts by weight, based on 100 parts of A, of a polyisocyanate B which is different from A and is present in a liquid form,
- in suspension in a dispersing agent C which is so chosen that A does not dissolve and B is so difficultly soluble therein that its adsorptive deposition on A is not prevented, and C is optionally thereafter removed, and

b) in a second stage,
- the polyisocyanate B which covers the surface of the polyisocyanate particles A is reacted with at least one polyvalent, isocyanate reactive compound D which is present in the liquid form in quantities of at least 0.5 equivalents of D per equivalent of B,
- optionally in suspension in a dispersing agent C to form a shell of A and D over A and

c) the dispersing agent C is optionally removed.

2. Process according to claim 1, characterised in that the dispersing medium of the first stage is removed and the reaction with component D is carried out in a different dispersing medium.

3. Process according to claims 1 or 2, characterised in that the substances used as dispersing medium C are aliphatic hydrocarbons and/or relatively long chained dialkylethers and/or plasticizers and/or relatively high molecular weight polyols.

4. Process according to claims 1 to 3, characterised in that the substances used as component A are aromatic polyisocyanates, preferably dimeric, trimeric diisocyanates and/or urea diisocyanates.

5. Process according to claims 1 to 4, characterised in that the substances used as component B are aliphatic, cycloaliphatic polyisocyanates and/or isocyanate prepolymers based on relatively high molecular weight polyhydroxyl compounds.

6. Process according to claims 1 to 5, characterised in that the substances used as component D are aliphatic or cycloaliphatic di- or polyamines, hydrazine, alkylhydrazines, N,N'-dialkylhydrazines and/or dihydrazide or polyhydrazide compounds and/or open chained monocyclic or bicyclic amidines or guanidines which are free from or have one or two isocyanate reactive hydrogen atoms.

7. Process according to claims 1 to 6, characterised in that compounds B and D react to form a polymer shell.

8. Enveloped solid, finely divided polyisocyanates with retarded reactivity obtainable by the process according to claims 1 to 7, optionally suspended in relatively high molecular weight polyols and/or relatively high molecular weight polyamines.

9. Use of the polyisocyanates according to claim 8 as polyisocyanate components for the preparation of polyurethanes.

10. Storage stable one-component reactive system containing polyisocyanates according to claim 8, relatively high molecular weight polyhydroxyl and/or polyamino compounds, optionally low molecular weight chain lengthening agents, optionally polyurethane catalysts and optionally conventional auxiliary agents and

additives.

## Revendications

1. Procédé de production de polyisocyanates solides en fines particules, à réactivité retardée par une enveloppe superficielle, caractérisé en ce que

a) dans une première étape
- on revêt en surface un polyisocyanate solide A en fines particules ayant un point de fusion au-dessus de 38°C et un diamètre des particules compris entre 0,5 et 200 µm,
- d'un polyisocyanate B différent de A, se trouvant sous la forme liquide, en une quantité de 0,05 à 50 parties en poids de B pour 100 parties de A,
- en suspension dans un agent C de mise en suspension, que l'on choisit de manière que A ne soit pas dissous et que B y soit si peu soluble que son dépôt par adsorption sur A ne soit pas empêché, à des températures inférieures au point de fusion de A, puis on élimine éventuellement C,

b) dans une seconde étape
- on fait réagir le polyisocyanate B revêtant la surface des particules de polyisocyanate A avec au moins un composé polyvalent D réactif vis-à-vis de groupes isocyanato, présent sous la forme liquide en quantités d'au moins 0,5 équivalent de D par équivalent de B,
- le cas échéant en suspension dans un agent C de mise en suspension, avec formation d'une enveloppe de B et de D autour de A, et

c) on élimine éventuellement l'agent C de mise en suspension

2. Procédé suivant la revendication 1, caractérisé en ce que le milieu de mise en suspension de la première étape est éliminé et la réaction avec le composant D est conduite dans un autre milieu de mise en suspension.

3. Procédé suivant la revendication 1 ou 2, caractérisé en ce qu'on utilise comme milieu C de mise en suspension des hydrocarbures aliphatiques et/ou des éthers de dialkyle à longue chaîne et/ou des plastifiants et/ou des polyols de poids moléculaire élevé.

4. Procédé suivant les revendications 1 à 3, caractérisé en ce qu'on utilise comme composant A des polyisocyanates aromatiques, de préférence des diisocyanates dimères ou trimères et/ou des urée-diisocyanates.

5. Procédé suivant les revendications 1 à 4, caractérisé en ce qu'on utilise comme composant B des polyisocyanates aliphatiques, cycloaliphatiques et/ou des prépolymères porteurs de groupes NCO, à base de composés polyhydroxyliques de poids moléculaire élevé.

6. Procédé suivant les revendications 1 à 5, caractérisé en ce qu'on utilise comme composant D des diamines ou des polyamines aliphatiques ou cycloaliphatiques, l'hydrazine, des alkylhydrazines, des N,N'-dialkylhydrazines et/ou des composés dihydrazidiques ou polyhydrazidiques et/ou des amidines ou guanidines à chaîne ouverte, monocycliques ou bicycliques portant 0, 1 ou 2 atomes d'hydrogène réactifs vis-à-vis de groupes NCO.

7. Procédé suivant les revendications 1 à 6, caractérisé en ce que les composés B et D réagissent avec formation d'une enveloppe de polymère.

8. Polyisocyanates solides en fines particules enveloppées, de réactivité retardée, pouvant être obtenus par le procédé selon les revendications 1 à 7, éventuellement en suspension dans des polyols de poids moléculaire élevé et/ou dans des polyamines de poids moléculaire élevé.

9. Utilisation des polyisocyanates suivant la revendication 8 comme composant polyisocyanate pour la production de polyuréthannes.

10. Système réactif à un composant stable à l'entreposage, contenant des polyisocyanates suivant la revendication 8, des composés polyhydroryliques et/ou polyaminés de poids moléculaire élevé, éventuellement des agents d'allongement de chaîne de bas poids moléculaire, éventuellement des catalyseurs pour polyuréthannes et, le cas échéant, des adjuvants et additifs classiques.